# EUROPÄISCHE PATENTANMELDUNG

(11) **EP 4 640 820 A1**
(43) Veröffentlichungstag der Anmeldung: **29.10.2025**
(21) Anmeldenummer: 24172289.1
(22) Anmeldetag: 24.04.2024
(51) Int. Cl.: C12M 1/42, B03C 5/00, B03C 5/02, C12M 1/00, C12N 13/00

(54) **SYSTEM UND VERFAHREN ZUM CHARAKTERISIEREN, SEPARIEREN, VERMEH-REN UND/ODER KRYOKONSERVIEREN MINDESTENS EINER BIOLOGISCHEN ZELLE**

(71) Anmelder: Fraunhofer-Gesellschaft zur Förderung der angewandten Forschung e.V., 80686 München (DE); Universität des Saarlandes, 66123 Saarbrücken (DE)
(72) Erfinder: ZIMMERMANN, Heiko, 66280 Sulzbach (DE); FUHR, Günter, 66280 Sulzbach (DE)
(74) Vertreter: Schiweck Weinzierl Koch Patentanwälte Partnerschaft mbB

(57) **Zusammenfassung**

Die Erfindung betrifft ein System zum Charakterisieren, Separieren, Vermehren und/oder Kryokonservieren mindestens einer biologischen Zelle, umfassend mindestens eine Vorrichtung aus mindestens einer biologischen Zelle und einem dielektrischen Trägerkörper, mindestens einen Behälter mit einem offenen Mündungsbereich und einem Bodenbereich, wobei die Vorrichtung in dem Behälter angeordnet ist, wobei der Bodenbereich mindestens eine erste Elektrode und mindestens eine zweite Elektrode zum Erzeugen eines elektrischen Wechselfelds innerhalb des Behälters aufweist, wobei der dielektrische Trägerkörper in einem elektrischen Wechselfeld mit einer ersten Wechselfrequenz eine schwächere negative dielektrophoretische Krafteinwirkung aufweist als in einem elektrischen Wechselfeld mit einer zweiten Wechselfrequenz, die höher als die erste Wechselfrequenz ist, und zumindest in dem elektrischen Wechselfeld mit der zweiten Wechselfrequenz eine stärkere negative dielektrophoretische Krafteinwirkung als ein Absolutwert einer dielektrophoretischen Krafteinwirkung einer einzigen biologischen Zelle in dem elektrischen Wechselfeld mit der zweiten Wechselfrequenz aufweist.

## Beschreibung

Die Erfindung betrifft ein System und ein Verfahren zum Charakterisieren, Separieren, Vermehren und/oder Kryokonservieren mindestens einer biologischen Zelle.

Die Tieftemperaturkonservierung von Zellen und Zellgruppen, insbesondere aber Organoiden für die Gewebeinduktion und Organentwicklung in vitro, ist inzwischen unabdingbar in nahezu allen Bereichen der Biotechnologie und Medizin geworden, stellt sie doch die einzige Möglichkeit dar, die Lebensprozesse mikroskopischer Objekte vollständig anzuhalten und zu einem beliebigen Zeitpunkt wieder in Gang zu setzen. Tiefgefroren sind Zell-Transporte über große Entfernungen, die Lagerung und Verfügbarhaltung vorbehandelter Zellen in Suspension und vieles mehr zum Standard in der Medizin, Pharmakologie und zur Voraussetzung für eine Vielzahl biotechnologisch ausgerichteter Industriezweige geworden. Die Kryokonservierung auf Temperaturen unterhalb -120°C, in der Regel gekühlt durch flüssigen Stickstoff (-196°C) oder elektrisch, wird in Zukunft noch weiter an Bedeutung gewinnen, da die inzwischen ebenfalls fortgeschrittene Stammzellforschung und -nutzung nach immer besseren und verfeinerten Klassifizierungen, insbesondere charakterisiertem Ausgangsmaterial, und nach dem Auftauen sofort erkennbaren Qualitätsmerkmalen verlangt. Die immer komplexer und auch finanziell aufwändigeren medizinischen Behandlungen (Immunzelltherapien, Organtransplantationen), biotechnologischen Verfahren der Gewebeinduktion für die Organogenese und Nutzung solcher Zellen für die Impfstoff- und Pharmakaentwicklung lohnen nur, wenn die Zellen nach dem Auftauen der erwarteten Qualität entsprechen. Ist das nicht der Fall, treten insbesondere im Bereich medizinischer Behandlungen erhebliche Gefahren, Risiken und Zusatzbelastungen für den Patienten auf.

Gerade in den ersten Minuten bis Stunden nach dem Auftauen befinden sich die revitalisierten Zellen nach einer Kryokonservierung in einem sich permanent verändernden Zustand. Das liegt daran, dass die Kryokonservierung kein natürlicher Vorgang ist und nur unter Nutzung spezieller Zusätze, insbesondere unphysiologischen Gefrierschutzmitteln, unter sehr definierten Randbedingungen erfolgreich und hochqualitativ durchgeführt werden kann. Die zugesetzten, obligaten Kryoprotektiva (wie Dimethylsulfoxid, Glycerin u.a.) müssen wegen ihrer unphysiologischen Konzentrationen und Wirkung möglichst rasch nach dem Auftauen entfernt werden, was einen Medienwechsel erfordert und einen zusätzlichen Stressor darstellt. Stark verkürzt lässt sich der Zustand lebender Zellen nach dem Auftauen, beginnend mit dem Zeitpunkt der Verflüssigung der Zellsuspension bei etwa -15 bis -5°C wie folgt zusammenfassen:
Die Zellmembranen sind durchlässiger als im Normalzustand, was zu Änderungen des Zellinnenmediums (Zytoplasma) als auch der Umgebungslösung (Kryo- bzw. Zellkulturmedium) führt.

Die unnatürliche Separierung der Zytoplasmabestandteile in kleine Eisdomänen und aufkonzentrierte Protein/Elektrolyt-Fraktionen im Zellinneren löst sich auf, ist jedoch nicht vollständig reversibel.

Die Zellen reagieren nach der kryobedingten Dehydrierung osmotisch und unterliegen daher mechanischen Kräften, insbesondere die Hüllmembranen, was sie permeabler macht.

Die Nukleation ist beim Einfrieren ungesteuert, d.h. rein stochastisch gewesen, woraus geschädigte Zellkompartimente resultieren.

Stoffwechselvorgänge werden angefahren, die im günstigen Fall zu den natürlichen Membranpotentialen und biochemisch ausbalancierten Zellreaktionen zurückführen.

Reparaturprozesse zum Austausch von gefriergeschädigten Makromolekülen und Membranelementen beginnen, in der Regel über Genaktivierungen, wofür Stunden bis Tage benötigt werden.

Die nicht sofort auswaschbaren Gefrierschutzmittel wirken unphysiologisch bis toxisch auf die Zellen.

Ein Teil der Zellen stirbt ab und die austretenden Zellsubstanzen belasten die noch vitalen Zellen.

Die in Pkt. a) bis h) genannten Probleme treten in unterschiedlicher Weise, aber bei allen Zellen auf. So sind beispielsweise Zellen, die sich beim Einfrieren gerade im Teilungszustand befanden, stärker belastet bzw. reagieren anders als ruhende Zellen, und auch zwischen den Zellarten und Zelllinien bestehen erhebliche Unterschiede, die z.T. noch nicht restlos verstanden sind. Entsprechend spricht man von erfolgreicher Kryokonservierung bei Überlebensraten zwischen 50 und 95%, wobei das Faktum des reinen Überlebens immer weniger als Qualitätskriterium ausreicht, weil man für die meisten Anwendungen Zellen eines ganz bestimmten Zustandes benötigt, d.h. so nahe wie möglich an den Zellzustand vor dem Einfrieren zurückkehren möchte. Dies ist bei allen bisherigen Kryoprozeduren nicht der Fall und stellt ein ungelöstes Problem mit großer Auswirkung auf die Biotechnologie und Medizin dar, auch wenn dies selten so und in der notwendigen Breite diskutiert, sondern verdrängt wird.

Da es keine Kryokonservierung ohne die in a) bis h) genannten Einschränkungen gibt, benötigt man quantitative Verfahren zur Erfassung von Gefrierschäden. Die bekannten Verfahren sind qualitativ, bei denen lediglich zwischen vitalen und nicht-vitalen Zellobjekten unterschieden wird, und reichen nicht aus. Es werden stattdessen Verfahren und Vorrichtungen benötigt, die es gestatten, Organoide und Zellaggregate nach dem Auftauen möglichst rasch, über einen Zeitraum von Sekunden bis zu Stunden oder Tagen zu charakterisieren und, wenn möglich, auch zu separieren.

Es existieren unzählige Zellcharakterisierungsverfahren, z.B. Fluoreszenzmarkierungen zur Unterscheidung zwischen lebend und tot, die in der Regel aber für die vitalen Zellen zum Gefrierstress noch weitere Belastungen hinzufügen oder die bereits erwähnten ja/nein-Antworten liefern. Andere Verfahren entnehmen Zellen aus der Suspension oder dem Zellverband, die dann zwar sehr detailliert analysiert (z.B. Zell-FACS), aber nicht immer weiterverwendet werden können. Solche noch so genau charakterisierten Zellen sagen zudem nichts aus über die Zellen der Probe, die nicht entnommen wurden, um die es aber gerade bei medizinischen und biotechnologisch-genetischen Anwendungen oft geht. Will man die Zellen oder auch Zellaggregate und Organoide einzeln erfassen und nicht als Durchschnittswert über viele Organoide, dann stehen nur drei generelle physikalische Mess- und Manipulationstechniken zur Verfügung: optische, akustische und elektrische. Entsprechende Applikationen sind seit über 40 Jahren immer wieder vorgestellt worden, haben aber keine breitere, universelle Lösung der genannten Probleme, insbesondere nicht bei der Kryokonservierung, erbracht.

Entsprechende Manipulationssysteme sind Laser-Tweezer, elektrische Hochfrequenz-Feldkäfige und Ultraschall-Fallen mit den dazugehörigen Charakterisierungen. Die wesentlichen Probleme bestehen vor allem darin, dass die physikalische Wechselwirkung maßgeblich durch die passiven optischen, elektrischen und akustischen Eigenschaften der biologischen Objekte bestimmt wird, deren Änderung man zwar in gewissen Grenzen erfassen kann, deren grundsätzliche Relationen zum Umgebungsmedium jedoch wenig Spielraum für Anpassungen lassen. Treten bei den Laser-Pinzetten in den Objekten beispielweise geringe Brechungsunterschiede zwischen den Kompartimenten auf, lassen sie sich nur schwer fangen und halten. Zudem befindet sich der Ort der höchsten Intensität (Fokus = höchste Belastung) gerade in dem gefangenen Objekt selbst, was die Anwendung zeitlich limitiert. Die dielektrophoretischen Kräfte (DEP) im Fall der elektromagnetischen Hochfrequenzapplikation, werden durch die Unterschiede der Dielektrizitätskonstanten (DK) und Leitfähigkeiten zum Umgebungsmedium (eine obligat leitfähige Zellkulturlösung) bestimmt, was die Freiheitsgrade ebenfalls deutlich einschränkt, weil die Zellkulturflüssigkeit maßgeblich den Verlauf der DEP-Spektren festlegt. Zeigt ein Objekt positive DEP, kann es nicht in der Bewegungsrichtung (hin zu den Elektroden) umgekehrt werde, analog bei negativer DEP (Abstoßung von den Elektroden), beispielsweise bekannt aus WO2023/16077711. Zudem induziert das Feld bei kHz-Frequenzen ein nicht unerhebliches Transmembranpotential, das die Zellmembranen belastet und E-Feldapplikationen über 5 kV/m sowie über Stunden oder Tage nicht erlaubt. Für die Ultraschallfallen gilt Ähnliches und wie bei den Laser-Tweezer-Systemen treten bei einer Parallelisierung bisher nicht zu beherrschende Nebeneffekte auf, ganz abgesehen von dem technischen Aufwand. Aus diesem Grund stehen parallelisierbare und technisch einfache Lösungen noch aus.

Hinzu kommt ein Problem bei der Kultivierung von Zellaggregaten und Organoiden in vitro. Derartige Gebilde wachsen mehr oder weniger kugelförmig radial nach außen sich vergrö-ßernd. Die Nährstoffe gelangen per Diffusion von der Oberfläche in das Innere des Zellverbandes. Im Unterschied zur Organogenese im Embryo tritt in vitro so gut wie nie eine Angiogenese auf, d.h. es wird kein Gefäßsystem ausgebildet, das die inneren Teile mit Nährstoffen versorgt und den Abtransport von Zellabprodukten erlaubt. Ab einer Größe von etwa 300 µm kommt es daher zu einer Nährstoffverknappung im Zentrum, die ab einem Durchmesser von ca. 500 µm zu Nekrosen und radial nach außen wirkendem Zelltod führt. Dem begegnet man über das Einbringen von Tubes, Fasern und porösen Materialien, die bioverträglich sind und die Diffusionsversorgung des Inneren über die genannte Größe des Zellaggregats hinaus erlauben. Neben diversen Hydrogelen sind das zahlreiche Matrix-Produkte für die 3D-Zellkultur wie Fibronectin-Tubes, Kollagenschwämme, Adhäsionsmoleküle oder Hyaluron-Gele. Diese künstlichen Elemente erweisen sich als sehr nützlich für die Stimulation und Steuerung der Differenzierung der Zellen, indem man ihre Oberflächen biofunktionalisieren kann.

Aufgabe der Erfindung ist es, ein Verfahren, eine Vorrichtung und/oder ein System zum Charakterisieren und/oder Separieren mindestens einer biologischen Zelle bereitzustellen, das Belastungen der untersuchten biologischen Zellen bei dielektrophoretischen Krafteinwirkungen reduziert oder vermeidet.

Die Aufgabe wird durch die Merkmale der unabhängigen Ansprüche gelöst. Vorteilhafte Weiterbildungen sind Gegenstand der abhängigen Ansprüche sowie der folgenden Beschreibung.

Bei einem System zum Charakterisieren und/oder Separieren mindestens einer biologischen Zelle, umfassend mindestens eine Vorrichtung aus mindestens einer biologischen Zelle und einem dielektrischen Trägerkörper, mindestens einen Behälter mit einem offenen Mündungsbereich und einem Bodenbereich, wobei die Vorrichtung in dem Behälter angeordnet ist, wobei der Bodenbereich mindestens eine erste Elektrode und mindestens eine zweite Elektrode zum Erzeugen eines elektrischen Wechselfelds innerhalb des Behälters aufweist, ist erfindungsgemäß vorgesehen, dass der dielektrische Trägerkörper in einem elektrischen Wechselfeld mit einer ersten Wechselfrequenz eine schwächere negative dielektrophoretische Krafteinwirkung aufweist als in einem elektrischen Wechselfeld mit einer zweiten Wechselfrequenz, die höher als die erste Wechselfrequenz ist, und zumindest in dem elektrischen Wechselfeld mit der zweiten Wechselfrequenz eine stärkere negative dielektrophoretische Krafteinwirkung als ein Absolutwert einer dielektrophoretischen Krafteinwirkung einer einzigen biologischen Zelle in dem elektrischen Wechselfeld mit der zweiten Wechselfrequenz aufweist.

Gemäß einigen Ausführungsbeispielen ist denkbar, dass die erste Elektrode und/oder die zweite Elektrode als eine Ringelektrode und/oder als eine Punktelektrode ausgebildet ist.

Gemäß einigen Ausführungsbeispielen ist denkbar, dass das die erste Elektrode und die zweite Elektrode in unterschiedlichen Abständen zu dem Mündungsbereich angeordnet sind.

Gemäß einigen Ausführungsbeispielen ist denkbar, dass das System mindestens zwei erste Elektroden und mindestens zwei zweite Elektroden aufweist.

Gemäß einigen Ausführungsbeispielen ist denkbar, dass die mindestens zwei ersten Elektroden abwechselnd ansteuerbar sind und/oder dass die mindestens zwei zweiten Elektroden abwechselnd ansteuerbar sind.

Gemäß einigen Ausführungsbeispielen ist denkbar, dass eine der mindestens zwei ersten Elektroden und eine der mindestens zwei zweiten Elektroden gleichzeitig ansteuerbar sind.

Gemäß einigen Ausführungsbeispielen ist denkbar, dass der Bodenbereich mindestens eine dritte Elektrode aufweist, die im Zentrum einer Quadrupolanordnung aus den mindesten zwei ersten Elektroden und den mindestens zwei zweiten Elektroden angeordnet ist.

Gemäß einigen Ausführungsbeispielen ist denkbar, dass die mindestens eine erste Elektrode und/oder die mindestens eine zweite Elektrode einzeln ansteuerbar ist.

Gemäß einigen Ausführungsbeispielen ist denkbar, dass die erste und zweite Elektrode Teil mindestens einer Quadrupolanordnung und/oder Oktupolanordnung von Elektroden in dem Behälter sind.

Gemäß einigen Ausführungsbeispielen ist denkbar, dass die erste und zweite Elektrode Teil einer Anordnung mit einem äußeren Quadrupol und einem inneren Quadrupol in dem Behälter sind.

Gemäß einigen Ausführungsbeispielen ist denkbar, dass der äußere Quadrupol zum Erzeugen eines elektrischen Wechselfelds mit einer ersten Feldstärke und der innere Quadrupol zum Erzeugen eines elektrischen Wechselfelds mit einer zweiten Feldstärke ausgebildet sind, wobei sich die erste Feldstärke und die zweite Feldstärke unterscheiden.

Gemäß einigen Ausführungsbeispielen ist denkbar, dass der innere Quadrupol und der äu-ßere Quadrupol um eine Mittelachse angeordnet sind und, vorzugsweise um 45°, unterschiedliche Drehwinkel zur Mittelachse aufweisen.

Gemäß einigen Ausführungsbeispielen ist denkbar, dass die erste Elektrode und/oder zweite Elektrode balkenförmig oder punktförmig ausgebildet ist.

Gemäß einigen Ausführungsbeispielen ist denkbar, dass sich die erste Elektrode und/oder die zweite Elektrode nichtparallel zu einer Erstreckungsrichtung des Behälters zwischen dem Bodenbereich und dem Mündungsbereich erstrecken.

Gemäß einigen Ausführungsbeispielen ist denkbar, dass alle ersten Elektroden des Behälters über eine erste elektrische Leitungsanordnung elektrisch miteinander verbunden sind und/oder alle zweiten Elektroden des Behälters über eine zweite elektrische Leitungsanordnung elektrisch miteinander verbunden sind.

Gemäß einigen Ausführungsbeispielen ist denkbar, dass die erste elektrische Leitungsanordnung und/oder die zweite elektrische Leitungsanordnung eine Isolatorschicht, vorzugsweise aus Siliziumdioxid, aufweist.

Gemäß einigen Ausführungsbeispielen ist denkbar, dass das System eine Vielzahl von Behältern aufweist, wobei in mindestens zwei Behältern jeweils mindestens eine Vorrichtung angeordnet ist.

Gemäß einigen Ausführungsbeispielen ist denkbar, dass die ersten Elektroden zumindest eines Teils der Vielzahl der Behälter mindestens eine erste gemeinsame Zuleitung aufweisen und/oder dass die zweiten Elektroden zumindest eines Teils der Vielzahl der Behälter mindestens eine zweite gemeinsame Zuleitung aufweisen.

Gemäß einigen Ausführungsbeispielen ist denkbar, dass die Behälter in Spalten und Reihen angeordnet sind, wobei die ersten Elektroden der Behälter mindestens einer Reihe oder mindestens einer Spalte über mindestens eine von der ersten gemeinsamen Zuleitung erste abzweigende Zuleitung elektrisch miteinander verbunden sind und/oder die zweiten Elektroden der Behälter mindestens einer Reihe oder mindestens einer Spalte über mindestens eine von der zweiten gemeinsamen Zuleitung zweite abzweigende Zuleitung elektrisch miteinander verbunden sind.

Gemäß einigen Ausführungsbeispielen ist denkbar, dass mindestens eine erste abzweigende Zuleitung mindestens einen Schalter zum elektrischen Trennen und Verbinden mit der mindestens einen ersten gemeinsamen Zuleitung aufweist und/oder dass mindestens eine zweite abzweigende Zuleitung mindestens einen Schalter zum elektrischen Trennen und Verbinden mit der mindestens einen zweiten gemeinsamen Zuleitung aufweist.

Gemäß einigen Ausführungsbeispielen ist denkbar, dass das System weiter mindestens eine Vorrichtung zum Bereitstellen mindestens einer elektrischen Wechselspannung mit einer einstellbaren Frequenz aufweist, die mit der ersten Elektrode und/oder zweiten Elektrode elektrisch Vorrichtungen ist.

Gemäß einigen Ausführungsbeispielen ist denkbar, dass das System weiter mindestens eine, vorzugsweise optische, Messeinrichtung zum Ermitteln einer Position mindestens einer Vorrichtung in mindestens einem Behälter aufweist, die an den Mündungsbereich des mindestens einen Behälters angeordnet werden kann, wobei die Behälter vorzugsweise auf einer beweglichen Transportvorrichtung angeordnet sind.

Gemäß einigen Ausführungsbeispielen ist denkbar, dass das System weiter ein Gerät zum Kryokonservieren mindestens einer Vorrichtung in mindestens einem Behälter aufweist.

Die Erfindung betrifft weiter ein Verfahren zum Charakterisieren und/oder Separieren mindestens einer biologischen Zelle, umfassen mindestens folgende Schritte: Bereitstellen mindestens eines Systems nach der vorangegangenen Beschreibung; Erzeugen eines ersten elektrischen Wechselfelds in dem Behälter mittels der ersten und zweiten Elektrode derart, dass die mindestens eine Vorrichtung in einem ersten Abstand von dem Bodenbereich entfernt angeordnet wird, wenn die mindestens eine biologische Zelle vital ist, und in einem zweiten Abstand von dem Bodenbereich entfernt angeordnet wird, wenn die mindestens eine biologische Zelle gestresst und/oder geschädigt ist, wobei der erste Abstand größer als der zweite Abstand ist, zum Separieren der mindestens einen biologischen Zelle; Ermitteln des Abstands der Vorrichtung von dem Bodenbereich zum Charakterisieren der mindestens einen biologischen Zelle.

Gemäß einigen Ausführungsbeispielen ist denkbar, dass das Verfahren weiter mindestens folgende Schritte aufweist: Erzeugen eines zweiten elektrischen Wechselfelds in dem Behälter mittels der ersten und zweiten Elektrode derart, dass die mindestens eine Vorrichtung in einem dritten Abstand von dem Bodenbereich entfernt angeordnet wird, wenn die mindestens eine biologische Zelle vital ist, und in einem vierten Abstand von dem Bodenbereich entfernt angeordnet wird, wenn die mindestens eine biologische Zelle gestresst und/oder geschädigt ist, wobei der vierte Abstand größer als der dritte Abstand ist; und Behandeln der Vorrichtung in dem Behälter, wobei das Behandeln vorzugsweise Kryokonservieren, Durchführen eines Substanztest, Durchführen von toxikologischen Tests und/oder Erfassen eines Zellwachstums umfasst.

Gemäß einigen Ausführungsbeispielen ist denkbar, dass das Verfahren zwischen dem Schritt: Erzeugen des zweiten elektrischen Wechselfelds, und dem Schritt: Behandeln, weiter mindestens folgenden Schritt aufweist: Entfernen von Vorrichtungen, die in dem vierten Abstand angeordnet sind, aus dem jeweiligen Behälter.

Gemäß einigen Ausführungsbeispielen ist denkbar, dass das Verfahren weiter mindestens folgenden Schritt aufweist: Bereitstellen mindestens einer kryokonservierten Vorrichtung in mindestens einem Behälter; und Erwärmen der mindestens einen kryokonservierten Vorrichtung und Erzeugen des ersten elektrischen Wechselfelds, vorzugsweise zumindest zeitweise mit einer Amplitude zwischen 4 V und 50 V, weiter vorzugsweise für weniger als 5 s.

Gemäß einigen Ausführungsbeispielen ist denkbar, dass die bereitgestellte kryokonservierte Vorrichtung an dem Bodenbereich angeordnet ist.

Gemäß einigen Ausführungsbeispielen ist denkbar, dass in mindestens zwei Behältern des Systems gleichzeitig ein elektrisches Wechselfeld mit gleicher Frequenz erzeugt wird.

Gemäß einigen Ausführungsbeispielen ist denkbar, dass das elektrische Wechselfeld dreidimensional und/oder rotationssymmetrisch ausgebildet ist und vorzugsweise ein elektrisches Gradientenfeld, weiter vorzugsweise ein nichtlineares elektrisches Gradientenfeld, aufweist.

Gemäß einigen Ausführungsbeispielen ist denkbar, dass die Vorrichtung aus dielektrischem Körper und der mindestens einen biologischen Zelle derart ausgebildet ist, dass sich die durch ein elektrisches Wechselfeld bei mindestens einer Wechselfrequenz erzeugte dielektrophoretische Krafteinwirkung auf die Vorrichtung verschwindet oder von einer negativen dielektrophoretischen Krafteinwirkung zu einer positiven dielektrophoretischen Krafteinwirkung ändert, wenn eine vorgegebene Anzahl an biologischen Zellen in der Vorrichtung überschritten wird.

Gemäß einigen Ausführungsbeispielen ist denkbar, dass der Schritt: Erzeugen eines ersten elektrischen Wechselfelds, dauerhaft durchgeführt wird, vorzugsweise bei einer Wechselfrequenz im Bereich von 100 kHz bis 1 GHz, weiter vorzugsweise im Bereich von 500 kHz bis 500 MHz, am meisten bevorzugt im Bereich von 5 MHz bis 100 MHz, wobei die mindestens eine biologische Zelle in dem dauerhaften ersten elektrischen Wechselfeld kultiviert wird.

Gemäß einigen Ausführungsbeispielen ist denkbar, dass die dauerhafte Durchführung des Schritts: Erzeugen eines ersten elektrischen Wechselfelds, beendet wird, wenn eine vorgegebene Anzahl an biologischen Zellen kultiviert wurde, insbesondere, wenn sich die durch das elektrische Wechselfeld erzeugte dielektrophoretische Krafteinwirkung auf die Vorrichtung von einer negativen dielektrophoretischen Krafteinwirkung zu einer positiven dielektrophoretischen Krafteinwirkung ändert.

Gemäß einigen Ausführungsbeispielen ist denkbar, dass das Verfahren weiter mindestens folgenden Schritt aufweist: Erfassen, vorzugsweise automatisiert, eines Wachstums der biologischen Zelle.

Gemäß einigen Ausführungsbeispielen ist denkbar, dass der Schritt: Ermitteln des Abstands, mit einer Genauigkeit im Bereich von 0,001 µm bis 100 µm, vorzugsweise von 0,01 µm bis 10 µm durchgeführt wird.

Gemäß einigen Ausführungsbeispielen ist denkbar, dass das erste elektrische Wechselfeld durch Variieren einer Wechselfrequenz ermittelt wird und/oder durch das Ermitteln eines Rotationsspektrums der mindestens einen biologischen Zelle, wenn das elektrische Wechselfeld als ein rotierendes elektrisches Wechselfeld ausgebildet ist.

Im Folgenden wird die Erfindung anhand einer beispielhaften Ausführungsform mittels der beigefügten Zeichnung beschrieben. Es zeigen:
- Figur 1: Schema zur allgemeinen Beschreibung der Organoid-Bewegung im Well;
- Figur 2: typische DEP-Spektren als Funktion der Frequenz des HF-Signals für tierische und humane Zellen;
- Figur 3: gemessenes DEP-Spektrum an Jurkat-Zellen und berechneter Verlauf zur Verdeutlichung der Genauigkeit der Modelle und Berechnungen und analoge Messungen;
- Figur 4: Typische DEP-Spektren und ihre maximale Beeinflussbarkeit durch Änderung eines Parameters;
- Figur 5: Kombinationen von Leitfähigkeit und Dielektrizitätskonstanten;
- Figur 6: Gesamtobjekt DEP-Spektren;
- Figur 7: Spektren eines in der Leitfähigkeit designtes und festgelegtes Bead mit einem Radius von 35 µm auf dem Zellen wachsen zu unterschiedlichen Besiedlungszeiten;
- Figur 8: Detailansicht eines DEP-Spektrums eines Organoids;
- Figur 9: DEP-Spektrum eines einschaligen Beads ohne Zellen;
- Figur 10: durch dielektrischen Körper verformtes DEP-Spektrum eines Organoids;
- Figur 11: Varianten und günstige Mikroelektrodenanordnungen in den Wells über die Feldverläufe;
- Figur 12: Quadrupolanordnungen mit umlaufendem Feld mit je zwei angesteuerten Elektroden;
- Figur 13: Quadrupol mit Zentralelektrode
- Figur 14: 8-Elektroden-Anordnung mit 1-Ebenen-Verdrahtung;
- Figur 15: Doppel-Quadrupolanordnung im HF-Wechselspannungsmodus;
- Figur 16: Elektrodenanordnungen und Feldlinien- bzw. Potential-Verläufe;
- Figur 17: Längliche Elektroden;
- Figur 18: Quadrupol-Elektroden-Konfiguration;
- Figur 19: DEP-Kraft-Trichter erzeugt mit vier planaren Elektroden an einem Wellboden;
- Figur 20: Anordnung von Elektroden in verschiedener z-Höhe;
- Figur 21: Zentral-Ring-Elektrodenkonfiguration in einer planaren Ausführung;
- Figur 22: Feldstärkeverteilung für zwei konische Ausführungen des Wellbodens;
- Figur 23: 2-Ring-Elektroden-Zentral-Elektrodensystem im Wellboden;
- Figur 24: Kultivierung von Zellaggregaten und Organoiden bei Langzeit-Feldapplikation;
- Figur 25: Anordnung des Ring-Zentral-Elektroden-Systems für ein 96-Well-Format;
- Figur 26: Variante der Anordnung aus Figur 25;
- Figur 27: Variante der Anordnung aus Figur 25;
- Figur 28: getrennte Einspeisung über mehrere Generatoren;
- Figur 29: Autofokus-System zum Ermitteln eines Abstands des Verbunds;
- Figur 30: alternative Messmethode des Abstands über einen eingekoppelten und am Boden reflektierten Messtrahl; und
- Figur 31: Gesamtsystem zur Kultivierung, Vermessung und Kryokonservierung von Zellkulturen in Multiwell-Formaten.

Als physikalisches Mess- und Manipulations-Prinzip werden hochfrequente elektrische Niedervoltsignale über planare und/oder dreidimensional angeordnete Mikroelektroden spezieller Ausprägung appliziert. Die Elektroden-Systeme sind so ausgeführt, dass die Objekte (Zellaggregate oder Organoide) über die Polarisation und die daraus resultierenden dielektrophoretischen Kräfte levitiert und sehr stabil schwebend in der Suspension des Wells ohne Wandberührung gehalten werden. Den Zellaggregaten werden dielektrische Mikrobestandteile (Beads, Tubes, oder ein Zentralköper bzw. eine umhüllende Kapsel) über die Kulturzeit zugegeben, die die Versorgung des Inneren der wachsenden Zellaggregate mit Nährstoffen, Differenzierungs- und Wachstums-Faktoren und anderen zell beeinflussenden Bestandteilen verbessern, gemäß einigen Ausführungsbeispielen aber in ihren dielektrischen Eigenschaften (Dielektrizitätskonstante und Leitfähigkeit) so festgelegt werden, dass sie zu einer Verschiebung der DEP-Spektren der Zellen (DielEktroPhoretische-Kraft als Funktion der Frequenz der elektrischen Anregungssignale) über den gesamten Bereich negativer bis zu positiver DEP führen. Die dielektrischen Eigenschaften dieser künstlichen Elemente werden gemäß einigen Ausführungsbeispielen gegenüber dem Stand der Technik so abgeändert, dass sie spezielle DEP-Spektren aufweisen. Im Wesentlichen solche, die die durch die Randbedingungen der Zellkultur weitgehend festgelegten DEP-Spektren der Zellen so verschieben, dass die nach der Kryokonservierung im E-Feld befindlichen Zellaggregate in Abhängigkeit von ihrer Vitalität über bestimmte Frequenzapplikationen in unterschiedlicher Höhe über den Elektrodensystemen in einem 3-dimensionalen Kraftfeld schweben und das auch im Bereich über 1 MHz tun, wo die Zellen an die Elektroden gezogen würden. Die dazu erforderliche Justage erfolgt einmal über die Wahl der applizierten Wechselspannungsfrequenz bzw. mehrere alternierend applizierte Frequenzen rein elektronisch und automatisiert, zum anderen über das passive dielektrische Design der Mikro-Elemente. Wie in den Beispielen gezeigt, können über die Kombination beider DEP-Spektren (des Zellaggregats und der dielektrischen Elemente) die vorher wenig beeinflussbaren Zell-DEP-Spektren nunmehr verschoben werden, so dass durch die Wahl der Charakterisierungsfrequenzen, die gleichzeitig auch die Levitationsfrequenzen sind, die ansonsten nicht verschiebbaren DEP-Spektren der Zellaggregate sogar zwischen starker negativer DEP (hohe Levitation) und ebenso kräftiger positiver DEP (keine Levitation und Anpressen an den Wellboden) hin und her verschoben werden können. Diese neuen künstlichen dielektrischen Elemente entsprechen nicht den bisher im Stand der Technik bekannten, können aber in gleicher Weise funktionalisiert und mit biologisch wirksamen Stoffen beladen werden. Gemäß einigen Ausführungsbeispielen handelt es sich um Elemente, die als Bead, Röhre, Hülle oder in anderer geometrischer Form ausgebildet werden können und deren DEP-Spektren sich bei niedrigen (< 1 MHz) und hohen Frequenzen (> 10 MHz) im Grundverlauf in etwa entgegengesetzt zum Verlauf der DEP-Zell-Spektren verhalten. Um das Spektrum möglicher Dielektrika für solche Elemente zu erweitern, werden gemäß einigen Ausführungsbeispielen nicht nur homogene, aus einem Material bestehende, sondern auch komplexer aufgebaute Elemente, wie ein- und mehrschalige Objekte sowie Mischungen aus verschiedenen dielektrischen Körpern genutzt. Aus der Summe der DEP-Kräfte des Zellaggregats und denen der künstlichen Elemente ergibt sich die resultierende frequenzabhängige DEP-Kraft des Gesamtsystems, die die Steighöhe der Zellaggregate im Kraftfeld bestimmt. Über die Verschiebung der Spektren oder auch Teile derselben in Bereiche neg. DEP ist es nun erstmalig möglich, den MHz-Bereich für eine Langzeitbefeldung zu nutzen, da hier nahezu keine induzierten Transmembrananteile durch das E-Feld mehr erzeugt werden.

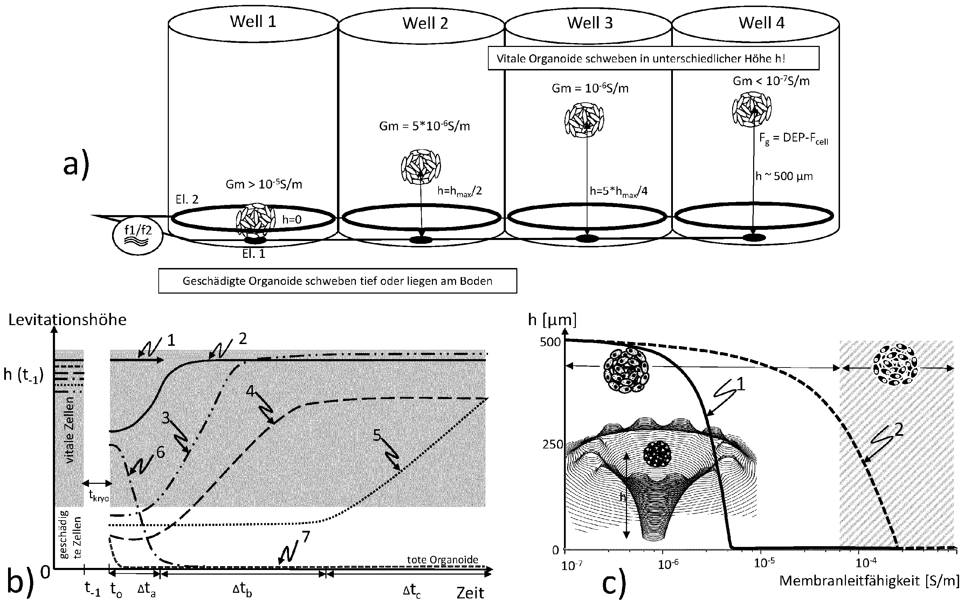

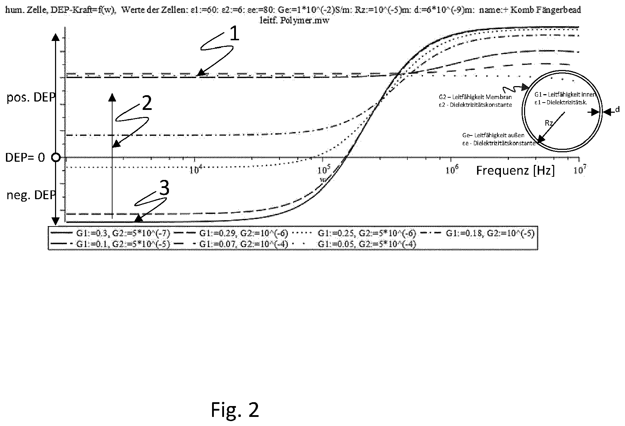

Das Verfahren der Charakterisierung und Manipulation der Zellaggregate würde bei einer Kryokonservierung (im Folgenden wird beispielhaft nur 1 Organoid pro Well beschrieben, es können aber auch mehrere sein) gemäß einigen Ausführungsbeispielen wie folgt ablaufen: Vor der Kryokonservierung wird eine Sondierungsapplikation einer oder alternierender elektromagnetischer Feldfrequenzen angelegt, die aus den bekannten DEP-Spektren abgeleitet wurden (beispielhaft 80 kHz). Über eine Frequenz- und Amplitudenanpassung wird eine möglichst hohe Steighöhe des vitalen Organoids im Well eingestellt und z.B. optisch, im Mikroskop oder anderweitig detektiert. Schwebt das Organoid nicht, besteht bereits eine Schädigung oder ein Wachstumsproblem vor der Kryokonservierung (Fig. 1). Über die Parallelisierung der Systeme in der Multiwell-Platte gilt diese Grundeinstellung nun für alle Wells und die darin befindlichen Organoide, muss also in der Regel nur an einem Well pro Platte ausgeführt werden. Alternativ kann sie an einem Modellobjekt (dielektrischer Musterkörper, in der Regel ein dielektrisches Eich-Bead) in einem Well oder als Computer-gespeicherte Voreinstellung absolviert werden. Diese individuelle Levitationshöhe (h(t-1), Fig. 1b) bildet den Vergleichswert für die zeitaufgelöste Charakterisierung nach dem Auftauen. Dafür eignet sich eine automatisierte Prozedur, da es sich um eine elektronische Steuerung handelt und die in Frage kommenden Frequenzen und Verschiebungen aus dem DEP-Summen-Spektrum grundlegend abgeleitet wurden oder vorher bestimmt werden können (vgl. Fig.2). Schweben die Organoide in verschiedenen Wells unterschiedlich hoch, dann sind auch ihre Eigenschaften bereits vor der Kryoprozedur unterschiedlich. Die Frequenz oder Frequenzen werden entsprechend den nachfolgenden Beispielen so gewählt, dass die Steighöhe zu einer Funktion der Membraneigenschaften und der Leitfähigkeit des Zytoplasmas der Zelle wird, denn das sind die entscheidenden Parameter zur Erfassung von Zellveränderungen (Permeabilitätserhöhung der Membran und Elektrolytverluste aus dem Zytoplasma). D.h., je höher ein Organoid schwebt, umso vitaler ist es (niedrige Membranpermeabilität und hohe Leitfähigkeit des Zytoplasmas), je niedriger, umso stärker weicht das Objekt vom erwarteten Zustand des Zellaggregats ab oder enthält nekrotisch bzw. absterbende Zellen (hohe Membranpermeabilität und verringerte innere Leitfähigkeit, Fig. 1b). Ein wesentlicher erfinderischer Anteil ist, dass mit Festfrequenzen gearbeitet wird und nicht die Erfassung der DEP-Spektren für die Charakterisierung erforderlich ist und, dass diese Festfrequenzen nicht die Maximalwerte, Nulldurchgänge oder Umkehrpunkte der DEP-Spektren nutzen.

Zum Einfrieren kann in dreierlei Weise verfahren werden:
Anwendung einer der üblichen Einfrierprozeduren, bei der die gesamten Multiwell-Platte auf Werte unter -120°C gekühlt wird (erfolgt auf einer Kühlplattform bei angeschaltetem Feld und in levitiertem Zustand). Die Organoide frieren in freier Lösung ein. Nachteilig daran ist, dass sie in einem relativ undefinierten Temperaturgradienten einfrieren, da die Kühlung vom Wellrand, insbesondere von dessen Boden aus erfolgt und Organoide in unterschiedlicher Höhe schweben.

Alternativ wird das HF-Feld abgeschaltet, das Organoid sinkt auf den Boden, wo es einem definierteren Temperaturgang (T =f(t)) ausgesetzt ist und schneller abkühlt.

Noch günstiger ist das Umschalten auf eine Frequenz f2 (beispielhaft 2 MHz), bei der die Levitation der Objekte gemäß einigen Ausführungsbeispielen invertiert wird, d.h. jetzt steigen die weniger vitalen und defekten Organoide auf, während die vitalen Aggregate auf die Bodenelektrode gezogen und dort angedrückt werden. Der engere Kontakt und die deutlich höhere Wärmeleitung der Metallelektrode und der gekühlte Boden lassen schnellere und definiertere Abkühlzeiten zu. Zudem besteht der Vorteil, noch vor dem Einfrieren die levitierten Organoide geringer Qualität abzusaugen und zu entfernen.

Beim späteren Auftauen der gesamte Well-Platte, kann das Mess- und Manipulations-E-Feld der Frequenz f1 bereits im gefrorenen Zustand angelegt werden, da über die Eisphase die Leitfähigkeit der Kulturlösung sehr gering ist und ein elektrisches Hochfrequenzfeld die Zellen kaum polarisiert. Im Moment der Verflüssigung steigt die Leitfähigkeit stark an, wodurch die Feldkräfte ebenfalls rasch zunehmen und das Organoid im Idealfall in die ursprüngliche Steighöhe (wie vor der Kryokonservierung) bewegen (Kurve 1 in Fig. 1c).

Vorteilhaft ist zudem, dass durch das Hochfrequenzfeld die Zellen im Inneren ein wenig erwärmt werden (~1°C), was den kritischen Auftauprozess bei der Kryokonservierung unterstützt, weil im Unterschied zur normalen Erwärmung von außen, hier die Wärme im Inneren der Zellen, also im leitfähigen Zytoplasma, erzeugt wird. Der Effekt kann gemäß einigen Ausführungsbeispielen durch kurzzeitige Erhöhung der Amplitude deutlich verstärkt werden (da eine quadratische Abhängigkeit der Erwärmung von der Amplitude vorliegt). Da die Zellen des Organoiden nach der Erwärmung die im Stand der Technik genannten Veränderungen (Pkt. a bis h) aufweisen, schweben sie je nach Grad der Abweichung zum Vor-Kryowert nun in verringerter Höhe, graduiert nach dem Grad der Kryo-Veränderungen (Fig. 1c).

In der Regel wird das Multiwellsystem wegen der vorgegebenen Auftau-Temperaturabläufe in einem Automaten aufgetaut und die Wells werden mit einem im Autofokus operierenden Mikroobjektiv mit Kamera Well für Well in kurzer Zeit abgefahren. Die Autofokuswerte liefern automatisch die Levitationshöhe. Das erfolgt wiederholt und dauert je nach Format (Zahl der Wells) Sekunden bis Minuten für einen Durchlauf. Entsprechend den Zeitkonstanten der jeweiligen Zellreparaturprozesse verändert sich die Levitationshöhe im Beobachtungszeitraum. Im Falle einer Stabilisierung der Membran (Verringerung der Permeabilität, Rückkehr zu Membranleitfähigkeiten von <10^-6 S/m) steigen die Organoide in den Wells auf und erreichen nach Minuten bis Stunden die Levitationshöhe vor der Kryokonservierung. Je länger dieser Prozess dauert, umso stärker kryo-beeinflusst waren die Zellen, was insbesondere für medizinische Anwendung von Bedeutung ist, wo man nur die besten Objekte für eine Therapie, eine Implantation oder einen Organersatz nutzen will. Organoide, die in ihrer Höhe verharren oder noch absinken, erweisen sich als nicht vital genug, um sich nach den Kryoschäden zu regenerieren, sind jedoch noch vital bzw. nur ein Teil der Zellen des Organoiden ist irreversibel geschädigt. Kultiviert man diese weiter, werden die vitalen Zellen sich vermehren und ggf. den Verlust ausgleichen, was entsprechend der Teilungsrate einige Tage in Anspruch nimmt. Derartige Organoide steigen dann über diesen längeren Zeitraum und langsamer auf, was im Wesentlichen der Zellvermehrung, einem weiteren wichtigen und erfassbaren Parameter in diesem Verfahren entspricht (Kurve 5, Fig. 1c). Gemäß einigen Ausführungsbeispielen ist dafür eine Dauerbefeldung über Stunden und Tage zweckmäßig und durch die Verschiebbarkeit der DEP-Zellspektren möglich geworden. Organoide, die nicht aufsteigen und zu Boden sinken, sind irreversibel geschädigt. Die Ausführungen verdeutlichen, dass die Steighöhe als Parameter zeitabhängige Datensätze pro Well mit umfangreichen Informationen zur Zellqualität, die Art der Kryobeeinflussung und die weitere Verwendbarkeit enthalten. Sie sind frei von subjektiven Bewertungen und können, wie die Beispiele zeigen, über Veränderung der applizierten Frequenzen gemäß einigen Ausführungsbeispielen noch weiter aufgelöst werden. Letztendlich kann man auch zwischen Membran- und Zytoplasma-Eigenschaften unterscheiden, wenn man die DEP-Spektren im oberen MHz-Bereich mit dem kHz-Teile vergleicht. Der Anschaulichkeit halber zunächst ein Schema zur Beschreibung der Organoid-Bewegung im Well (Fig.1) gemäß einigen Ausführungsbeispielen.

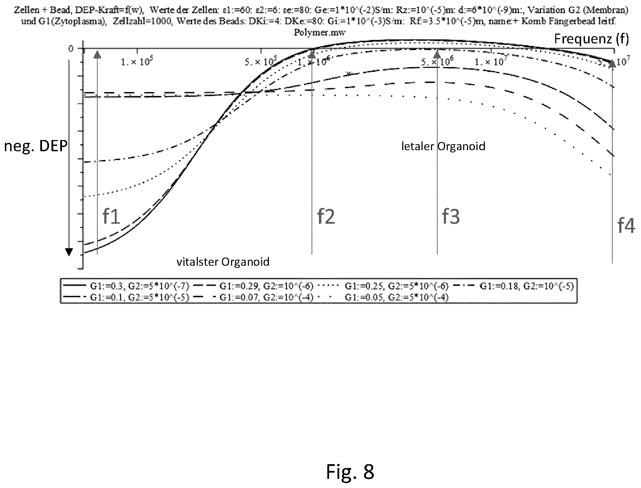

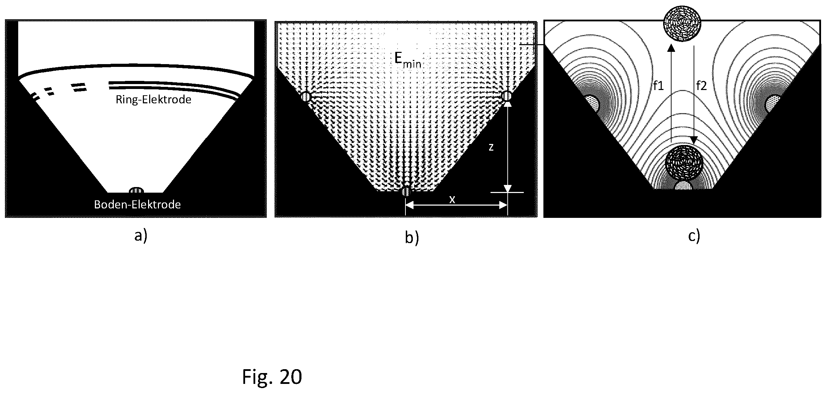

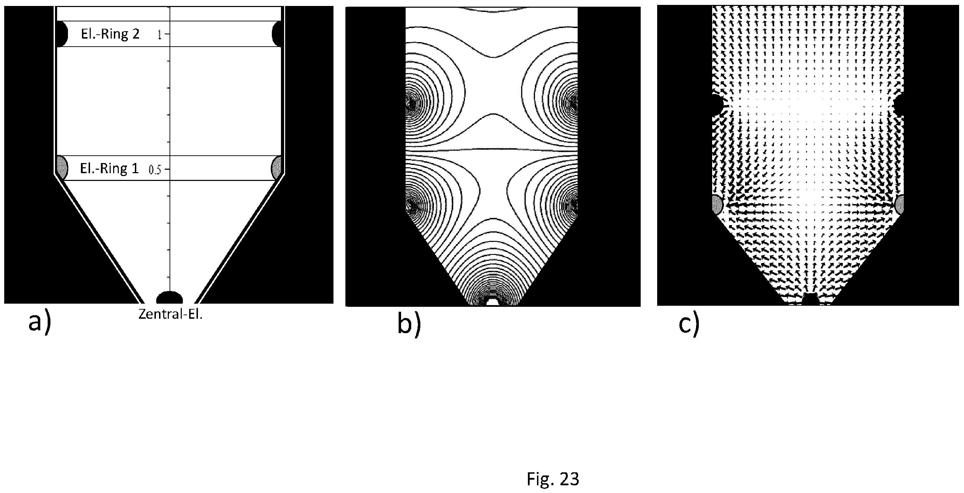

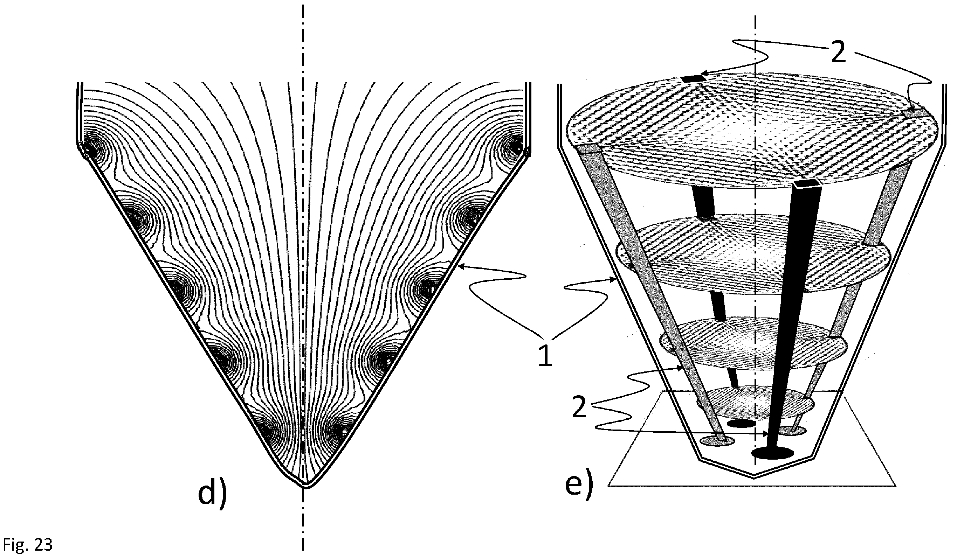

Fig. 1: a) Veranschaulichung der Beziehung zwischen Levitation (Höhe h) im Zentralbereich des Wells in Abhängigkeit von den Membraneigenschaften (Gm) der Zellen. Die Anregung erfolgt über jeweils ein Ring-Zentral-Elektrodensystem (vgl. auch Fig. 20 und 23) mit einem HF-Generator einstellbarer Festfrequenzen (hier f1 und f2, vgl. Fig. 8).

In b) sind die unterschiedlichen Fälle der Levitations-Zeit-Verläufe nach einer Kryokonservierung über den Zeitraum tkryo aufgetragen. Der Zeitpunkt t-1 gibt den Vergleichswert h vor der Kryokonservierung an. Organoide im grauen Bereich sind als vitale Zellen mit unterschiedlicher Membranleitfähigkeit anzusehen. Die darunter im hellen Bereich liegenden Zellaggregate sind geschädigt, nekrotisch oder bereits abgestorben. (1) wäre der ideale Verlauf nach der Kryokonservierung, keine Veränderung, der allerdings kaum auftritt. (2) Die Zellen regenerieren innerhalb der Periode Δta , d.h. innerhalb von Minuten bis zu einer Stunde. Bei (3) und (4) erfolgt das über die Periode (Δta + Δtb), was auf Reparaturprozesse mit Genaktivierung schließen lässt (Stunden bis zu einem Tag). Kurve (5) zeigt einen Organoid bei dem viele Zellen irreversibel geschädigt wurden, bei dem die überlebenden Zellen über Tage eine Neubesiedlung vornehmen. In (6) und (7) sind die Verläufe für absterbende oder bereits durch die Kryokonservierung irreversibel geschädigte Organoide zu sehen.

In c) wird nochmals das zu lösende Problem verdeutlicht: Kurve (1) zeigt die Levitationshöhe von Organoiden in Abhängigkeit von der Membranleitfähigkeit der Zellen (der schraffierte Teil markiert den Bereich geschädigter Zellaggregate). Durchaus noch vitale und regenerationsfähige Zellen mit einer Membranleitfähigkeit von 5*10^-6 S/m würden bereits auf dem Boden des Kraftfeldtrichters liegen. Die Kurve (1) soll daher gemäß einigen Ausführungsbeispielen in Richtung und in die Position der Kurve (2) verschoben werden, was gemäß einigen Ausführungsbeispielen über wohlabgestimmte dielektrischen Körper erfolgt (siehe weiter unten).

Gemäß einigen Ausführungsbeispielen kann die nichtlineare Steighöhenabhängigkeit von der applizierten Amplitude der Wechselspannungen über die Elektrodenanordnung in gewissen Grenzen verbessert, d.h. linearisiert werden (vgl. nachfolgende Beispiele des Feldgradienten in der Mittelachse des Wells).

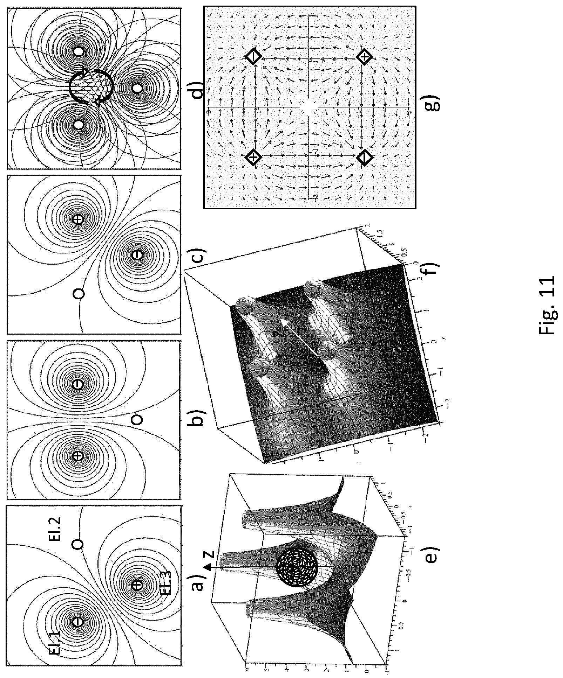

Da sehr leitfähige Zellkulturlösungen (im Bereich von 10^-2 bis 10^-1 S/m) obligatorisch sind, tritt ein technisches Problem bei der Parallelisierung auf, was nachfolgend am Beispiel der 384-Well-Platte verdeutlicht werden soll. In jedem Well befindet sich eine Mikroelektrodenanordnung, die aus mindesten zwei Elektroden besteht und über die das E-Feld in der Lösung erzeugt wird, das dann die Objekte polarisiert. Aus der Wechselwirkung der Polarisationsladungen an den Grenzflächen der Dielektrika ergibt sich die DEP-Kraft, die auf das Objekt wirkt. Wie in Fig. 1a gezeigt, wäre die einfachste Lösung eine Parallelschaltung der Well-Elektrodensysteme (hier eine Punkt- und eine Ring-Elektrode pro Well). Abgesehen von der Kapazität, die allerdings sehr gering ist, bedeutet das eine Parallelschaltung der Eingangswiderstände der Well-Elektrodensysteme am Ausgang des Hochfrequenzgenerators. Will man nicht in technisch schwierig zu handhabende niedrige Eingangswiderstandsbereiche kommen, muss man den Widerstand von Elektrode 1 über die Zellkulturlösung zur Elektrode 2 so hoch wie möglich halten, da sich der Gesamteingangswiderstand der Multiwell-Platte durch jede Parallelschaltung verringert. Hier ist also weniger die Impedanz, sondern deren Realteil, der rein ohmsche Widerstand=1/Leitfähigkeit entscheidend. Das bedeutet, die Elektrodenflächen, die mit der Kulturlösung in Verbindung stehen, möglichst klein zu halten, aber groß genug, um ausreichend E-Feld einzukoppeln, so dass eine effektive DEP-Kraft auf die Organoide wirkt. Gemäß einigen Ausführungsbeispielen hat die hier vorgestellte Lösung der Kombination von dielektrischen Elementen und auf ihnen oder in ihnen wachsenden Zellen den Vorteil, dass ein Teil des applizierten Feldes Kräfte auf die künstlichen Elemente ausübt, so dass die Belastung der Zellen gegenüber dem Stand der Technik verringert werden kann (halbiert oder darunter). Entsprechend effektive Elektrodenanordnungen werden weiter hinten dargestellt (Fig. 11 bis 23). Messungen zeigen, dass dennoch der Widerstand solcher Elektrodenanordnungen aufgrund der leitfähigen Zellkulturlösungen im unteren kOhm-Bereich liegt. Das scheint bei günstigen Hochfrequenzgenerator-Ausgängen mit 50 Ohm Belastbarkeit ausreichend hoch, eine Parallelschaltung von 384 Systemen würde jedoch zu einem Belastungswiderstand am Ausgang des Generators im einstelligen Ohm-Bereich führen, was zum Zusammenbruch der Generatorsignale führt, ganz abgesehen von der Leistung, die elektronisch zur Verfügung gestellt werden müsste, um die Signale aufrecht zu erhalten. Da in diesem Verfahren Frequenzen zwischen 100 kHz bis 100 MHz appliziert werden, würden die genutzten Rechtecksignale bei hohen Frequenzen stark verschliffen und mehr und mehr zu sinusförmigen Signalverläufen sich stark verringernder Amplitude führen. Die Parallelschaltung der Elektroden-Wellsysteme muss zudem auch über die Leitungstheorie der Elektrotechnik betrachtet werden. Wie bei einer Fernleitung, kommt nahe der Einspeisung mehr Leistung an als beim letzten Well, was ebenfalls nicht tolerierbar ist.

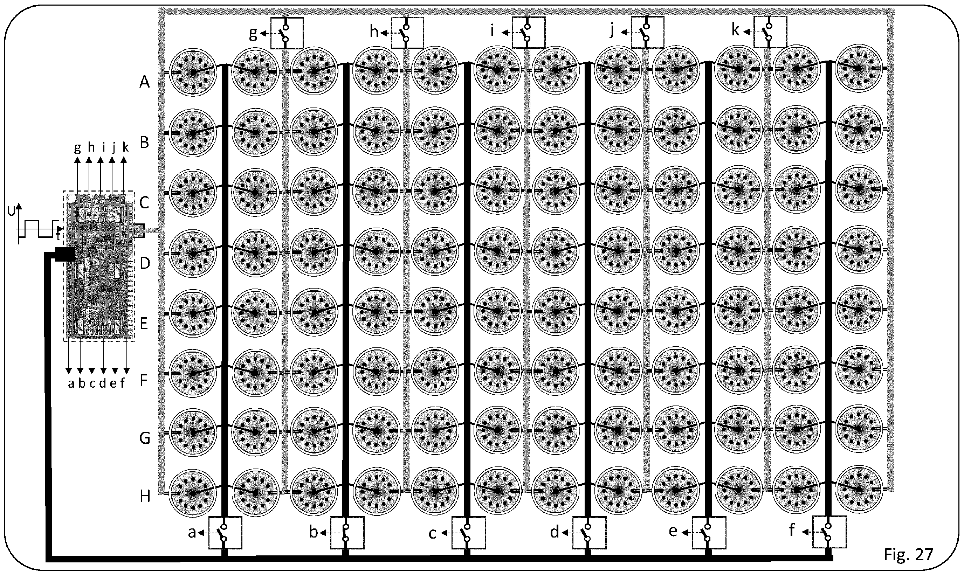

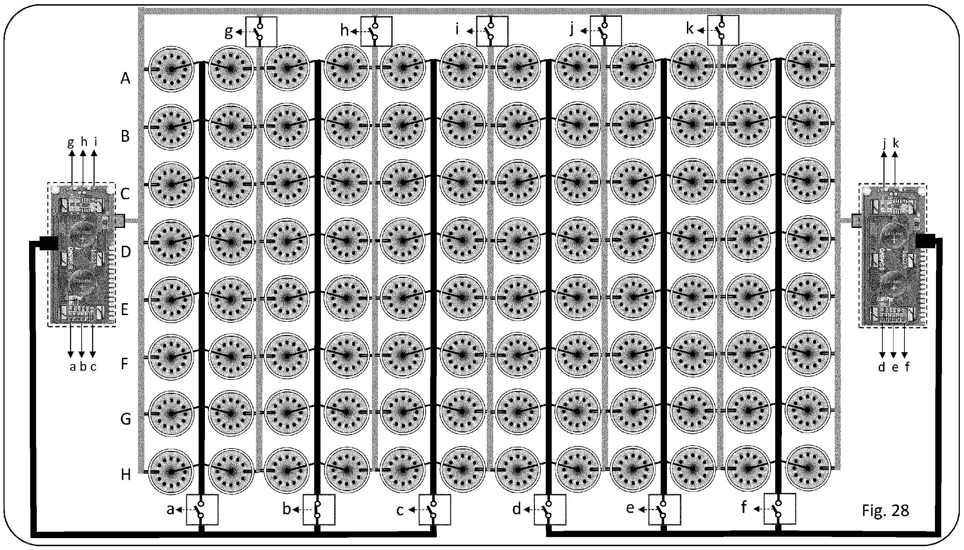

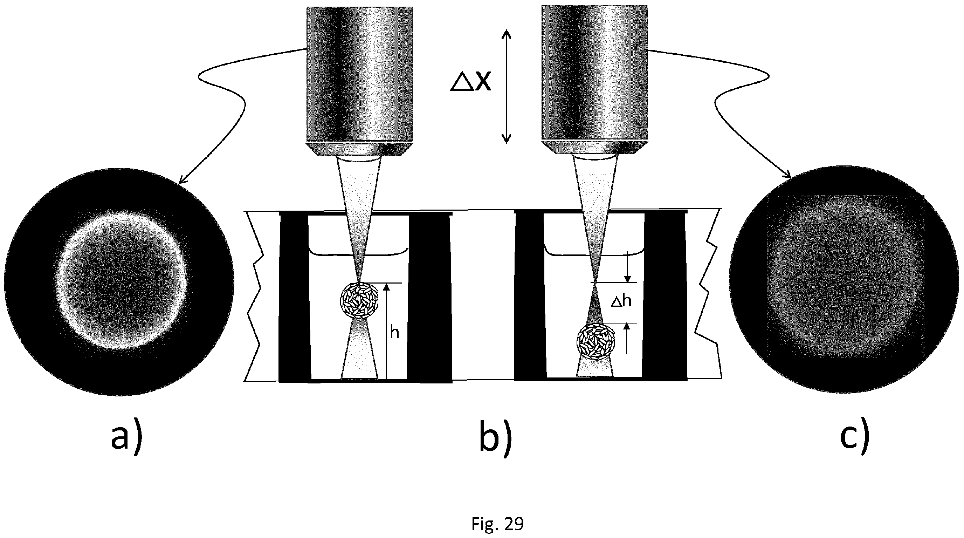

Gemäß einigen Ausführungsbeispielen wird das Problem auf zweierlei Wegen gelöst: Einmal durch mehrfache Einspeisung von verschiedener Seite der Well-Platte (vgl. Fig. 29), zum anderen durch Beschaltung von jeweils nur einem Teil der Well-Elektroden der Platte (vgl. Fig. 27 und 28). Das kann so weit getrieben werden, dass immer nur das Well angesteuert wird, in dem gerade die Levitationshöhe des Organoiden bestimmt wird.

Bliebe noch die Verringerung der Wärmeproduktion im Wellsystem. Einmal wird sie gut abgeleitet über die große Wellfläche, zum anderen lässt sich bei Organoiden der folgende Effekt nutzen: Die Sedimentationskräfte, die auf die Organoide in der Kulturlösung wirken, sind aufgrund der sehr ähnlichen Dichte gering, sie sedimentieren im Sekunden-Bereich, d.h. sehr langsam in Richtung Well-Boden. Die Organoide müssen daher nicht immer mit voller Feldkraft für den Messvorgang angehoben werden, sondern nur, wenn sie vermessen werden. Die applizierte Amplitude der HF-Signale kann daher an den meisten Wells stark reduziert oder die Felder können sogar an- und abgeschaltet werden.

Wie sich bei Messungen zeigte, erlaubt erst die Gesamtheit der genannten Kombinationen die praktische Nutzung und die Lösung der Aufgabe einer Charakterisierung von kryokonservierten Zellen in Multi-Well-Formaten.

Zum allgemeinen Verständnis sind die aus dem Stand der Wissenschaft seit langem vermessenen und modellierten DEP-Zellspektren von Bedeutung. Fig. 2 zeigt typische DEP-Spektren als Funktion der Frequenz des HF-Signals für tierische und humane Zellen (induzierte pluripotente Stammzellen oder andere Stammzellen). Im Grunde zeigen aber alle Zell-Spektren in etwa qualitativ diesen Verlauf, weil die Außenbedingungen (Kulturmedium, eine leitfähige wässrige Flüssigkeit) diese Kurvenform weitgehend determinieren.

Fig. 2: DEP-Spektren tierischer und humaner Zellen. Das Kern-Problem besteht darin, dass die DEP-Kräfte in Abhängigkeit von der Frequenz sowohl negativ als auch positiv sein können. Negative Werte bedeuten, Zellen und Zell-Aggregate werden von den Elektroden abgestoßen, d.h. in Bereiche der E-Feld-Minima gedrückt, positive Werte, sie werden an die Elektroden gezogen (Feld-Maxima, vgl. Beschriftung der Ordinate). Wie in der Aufgabenstellung erläutert, befinden sich die vitalen Zellen bis zu etwas mehr als 100 kHz im negativen Bereich, was für die Levitation erforderlich ist, aber eben auch immer noch vitale Zellen mit etwas geringerer Leitfähigkeit der Membran im positiven DEP-Bereich, in dem all das, was zur Lösung der Aufgabe gebraucht wird, nicht umsetzbar ist. Mit 1 sind die DEP-Verläufe für schwer kryogeschädigte Zellen, mit dem Pfeil 2 die zunehmende Schädigung und mit 3 völlig intakte, vitale Zellen bezeichnet. Zudem tritt bei hohen Frequenzen oberhalb 100 kHz ausschließlich positive DEP auf, so dass auch dieser Bereich, der kaum mehr eine Membranpotential-Belastung erzeugt, nicht genutzt werden kann. In 7 Kurvenverläufen wurde die physiologische Situation einer zunehmenden Membranleitfähigkeit bei parallelem Verlust von Elektrolyten aus dem Zytoplasma, wie sie vielfach gemessen wurden, zusammengefasst (Schädigung einer Zelle). Das zweite Problem besteht darin, dass man diese Spektren nur über die Umgebungsflüssigkeit (Kulturmedium) im allgemeinen Kurvenverlauf beeinflussen kann. Da diese vom Wasser dominiert wird, steht auch die Dielektrizitätskonstante mit ~ 80 fest. Die hohe Leitfähigkeit kann nur um etwa 1 Zehnerpotenz verschoben werden, da die Zellen die verschiedensten Ionen im Außenmedium benötigen. Mit den Zellen allein, besteht also kein Freiheitsgrad mehr, um die Aufgabe einer höhenabhängigen Charakterisierung von Zellen zu lösen.

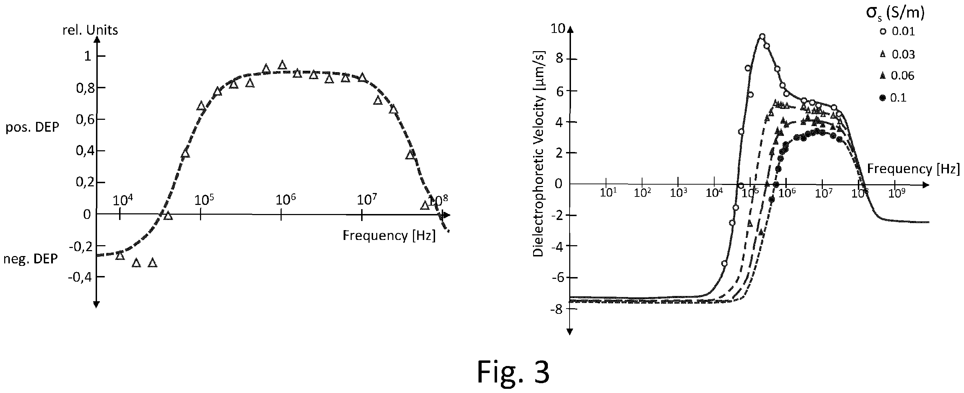

Zur Verdeutlichung des Verfahrens und Verständnis der dazu erforderlichen Vorrichtungen wird im Folgenden auf Modellierungen zurückgegriffen, obwohl auch Messungen vorliegen. Vielfach publizierte DEP-Messungen belegen, dass die benutzten Modelle (die immer auch nachfolgend angegeben werden) in sehr guter Übereinstimmung mit den Messwerten stehen (deutlich über 95%). Beispielhaft sei eine Abbildung und Literatur-Stelle zitiert (Fig. 3), so dass im Weiteren auf Messwerte verzichtet werden kann.

Fig.3: Links - gemessenes DEP-Spektrum an Jurkat-Zellen und berechneter Verlauf zur Verdeutlichung der Genauigkeit der Modelle und Berechnungen Rechts - analoge Messungen.

Für die gemäß einigen Ausführungsbeispielen durchgeführte Verschiebung/Justage der Zell-DEP-Spektren über dielektrische Mikroelemente wird zweierlei benötigt:
Dass ihr DEP-Spektrum es erlaubt, die Zell-Spektren für alle Membran- und Zytoplasma-Kombinationen der passiven elektrischen Eigenschaften nach Belieben zwischen positiver und negativer DEP zu verschieben.

Dass dafür wenigsten ein Parameter zur Verfügung steht, so dass man diese Verschiebung durch spezielle Materialien festlegen, aber auch in einem möglichst breiten Bereich voreinstellen kann (z.B. über vorgefertigte Beads). Das muss für den gesamten gezeigten Frequenzbereich zutreffen.

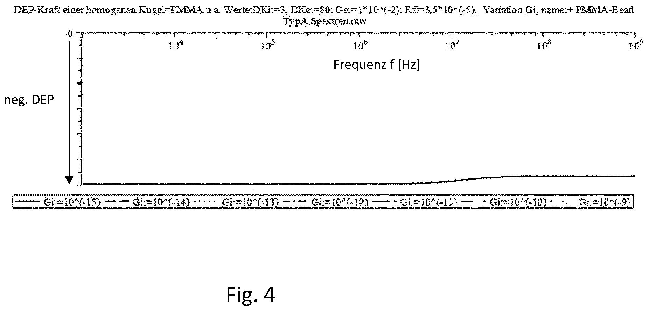

Typische DEP-Spektren und ihre maximale Beeinflussbarkeit durch Änderung eines Parameters sind in Fig. 4 beispielhaft gezeigt. Zwar liegen sie alle im negativen DEP-Bereich, aber so stark, dass sie bei Zugabe die Zell-DEP-Spektren weit in den neg. DEP-Bereich ziehen und so gut wie nicht graduell eingestellt werden können und wenn, dann nur im ungünstigen Hochfrequenz-bereich (> 10 MHz). Das bedeutet aber, alle Zellaggregate und Organoide schweben weit oben und eine quantitative Unterscheidung wäre nicht möglich.

Fig. 4: Homogenes Bead aus PMMA, Polyamid, Polyurethan, SiO2 oder Al2O3. Das alles sind Materialien mit extrem niedriger Leitfähigkeit (Gi=10^-12... 10^-15 S/m) und niedriger DK=3...6. Damit ebenfalls keine Beeinflussbarkeit der Spektren über Veränderungen Gi oder die Dielektrizitätskonstante möglich.

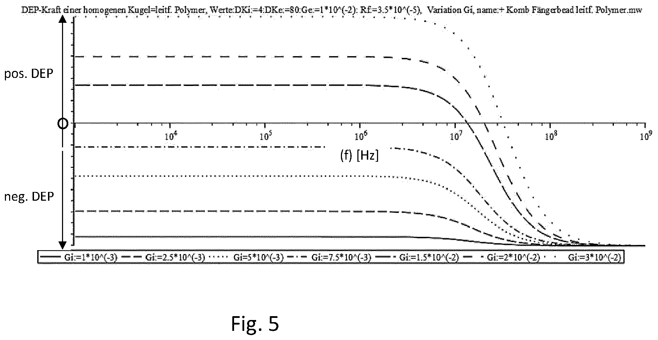

Wie Fig.4 exemplarisch belegt, lassen sich die gegenwärtig benutzten Materialien zur Strukturierung und Versorgung von Organoiden nicht in der Weise nutzen, wie es entsprechend der erfinderischen Lösung erforderlich ist. Es werden stattdessen in weiten Bereichen variierbare dielektrische Eigenschaften mit in etwa inversem Verlauf zu den DEP-Zellspektren (Fig. 2) benötigt. Ein bevorzugter Parameter ist dabei die innere Leitfähigkeit (Gi), da diese durch Zugabe von leit-fähigen Feststoffanteilen oder geladenen Molekülgruppen und molekularen Dipolen, z.B. in ein Polymer, realisierbar ist. Kombinationen von Leitfähigkeit und Dielektrizitätskonstanten, wie sie benötigt werden zeigt Fig. 5.

Fig. 5: Wie die DEP-Spektren verdeutlichen, werden Leitfähigkeiten benötigt, die im Bereich von 10^-3 bis 3*10^-2 S/m liegen. Das ist als Feststoff-Mikrokörper durch Zumischung z.B. von leitfähigen Nanopartikeln, Carbonfasern, leitfähigen Molekülresten usw. möglich, wie sie etwa in Leitpasten in der Halbleitertechnologie und in der Polymer-Chemie verwendet werden. Von besonderer Bedeutung ist der im Bereich von Frequenzen über 4 MHz abfallende Kurvenverlauf, da nur so die stark in die pos. DEP wechselnden Kräfte auf Zellen kompensiert werden können (in etwa inverser Verlauf der DEP-Spektren, wenn man einmal vom GHz-Bereich bei Zellen absieht).

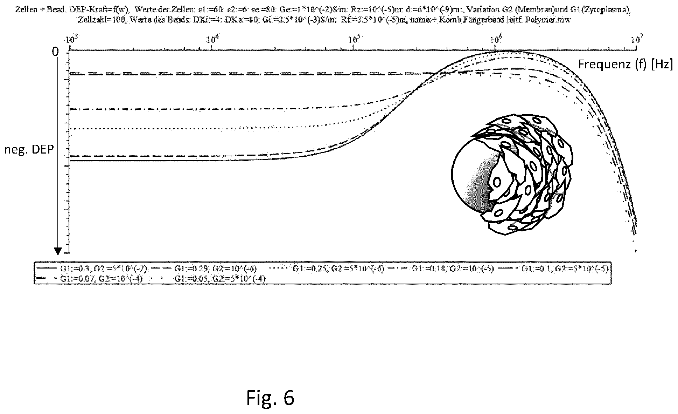

Fig. 6: DEP-Spektren eines moderat leitfähigen Polymer-Beads (2,5*10^-3 S/m) von 35 µm Radius auf dem Zellen wachsen und einen Organoid (aus 100 Zellen) geformt haben (Zellbesiedlung hier nur zur Hälfte rechts dargestellt, damit das zentrale Bead erkennbar ist), in Abhängigkeit von der Vitalität der Zellen (Kombination aus den Eigenschaften der Membran und des Zytoplasmas, vgl. Box). Die gesamten Bereiche pos. DEP der Zellen (vgl. Fig. 2) werden durch das Bead wie erforderlich zur Lösung der erfinderischen Aufgabe in den Bereich neg. DEP verschoben und insbesondere der MHz-Bereich in der Abstoßungskraft von den Elektroden verstärkt.

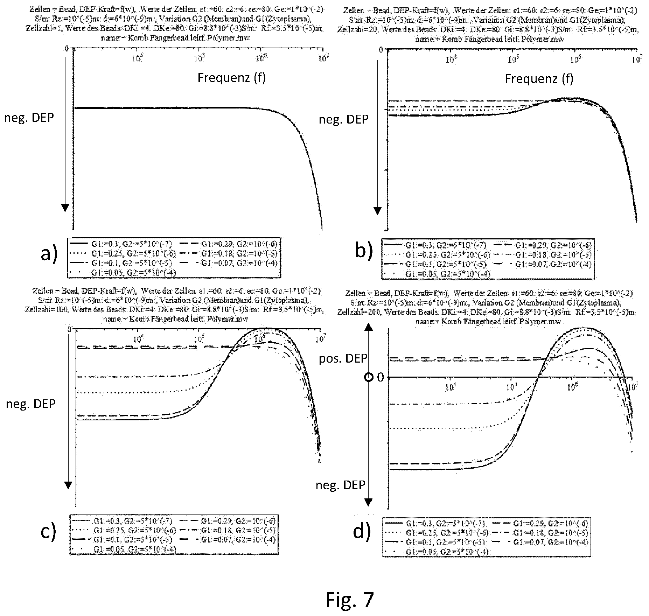

Bevor auf weitere Ausführungsbeispiele dielektrischer Mikroelemente und die beschriebene Kryoprozedur eingegangen wird, muss gezeigt werden, in welchem Zustand des Bewuchses im Spektrum des Gesamtsystems überhaupt etwas über die Zellen und im Speziellen quantitativ auch ihre Vitalität erkennbar sein wird. Das wird deutlich, wenn man die beiden Extremfälle, a) nur 1 Zelle auf dem größeren Bead und b) 1000 Zellen auf dem nun sehr kleinen Bead-Volumenanteil betrachtet (Fig. 7).

Fig. 7: Die Spektren a) bis d) zeigen ein in der Leitfähigkeit designtes und festgelegtes Bead mit einem Radius von 35 µm auf dem Zellen wachsen zu unterschiedlichen Besiedlungszeiten: in a) 1 Zelle auf dem Bead, die erwartungsgemäß so gut wie keinen Einfluss auf das Gesamtsystem hat. In b) handelt es sich um 20 Zellen, was einer noch nicht geschlossenen Oberflächenbedeckung entspricht. Trotzdem erkennt man bereits die Aufspaltung der Spektren in Abhängigkeit von den Membran-/Zytoplasma-Eigenschaften der Zellen im Bereich der neg. DEP-Kräfte (Abstoßung von den Elektroden). In c) ist die Besiedlung im optimalen Bereich für diese Wahl der Parameter und Größe des Beads gezeigt. Die Oberfläche ist mit 100 Zellen bereits in mehreren Zellschichten überwachsen (Organoid). Die Spektren befinden sich gemäß einigen Ausführungsbeispielen vollständig im Bereich neg. DEP-Kräfte. In d) handelt es sich nun um einen Bewuchs mit 200 Zellen und das resultierende Spektrum wird mehr und mehr von den Zellen dominiert, wodurch bei geschädigten Zellen und im oberen Frequenzbereich wieder positive DEP auftritt. Dennoch würden im kHz-Bereich die Objekte mit vitalen Zellen in unterschiedlichen Levitationshöhen schweben, während geschädigte Organoide auf die Elektroden gezogen werden.

Fig. 7 verdeutlicht, dass es sich bei der frequenzabhängigen Polarisation von Bead und Zellen um Volumenkräfte handelt und dadurch in einem sehr breiten Besiedlungsbereich die gewünschten Effekte der differenzierten Levitation auftreten und genutzt werden.

Auf der Basis dieser Spektren lässt sich nun verdeutlichen, worin der Vorteil solcher Bead-Zell-Kombinationen besteht, insbesondere welche Informationen und Manipulationen/Separationen bei Änderung der Frequenz der applizierten HF-Felder erzielt werden können:
Wie bereits in den Zellspektren in Fig. 2 erkennbar war, gibt es einen Kreuzungsbereich der Spektren im oberen kHz-Bereich. Bei niedrigeren Frequenzen (< 400 kHz) zeigen die vitalen Zellen die größte negative DEP und die geschädigten bzw. toten Zellen die geringsten Werte. Entsprechend werden sich die Zell-Bead-Komplexe im Feld in der Levitationshöhe verhalten (vgl. Fig. 1, vitale Zellen führen zur größten Levitationshöhe). Im Frequenzbereich oberhalb 500 kHz dreht sich die Reihung der Spektren um. Auch hier führen die vitalen Zellen zur stärksten DEP-Kraft, die nun allerdings positiv ist. Durch das Bead wird nun aber die gesamte Kurvenschar gemäß einigen Ausführungsbeispielen in den negativen DEP-Bereich gezogen, wodurch sich tatsächlich eine invertierte Anordnung der Zell-Bead-Komplexe entsprechend ihrer Vitalität und Levitation ergibt. In beiden Frequenzbereichen werden sie nun von den Elektroden abgestoßen. Im niedrigen Frequenzbereich schweben wie bisher die vitalsten Organoide ganz oben, im oberen Frequenzbereich jedoch die letalen. In Fig. 8 ist zur Erläuterung der Nutzung verschiedener Frequenzen der Frequenzausschnitt zwischen 5*10^-4 Hz und 5*10^-7 Hz bei einer Besiedlung mit 1000 Zellen dargestellt.

Fig. 8: Legt man an ein Elektrodensystem (siehe weiter hinten) die Frequenz f1 (60 kHz) an, ordnen sich die Organoide entsprechend ihrer Vitalität an (die vitalen Organoide schweben am höchsten in abnehmender Reihung bis zu letalen ganz unten im Well). Will man beispiels-weise vor dem Einfrieren die geschädigten Organoide entfernen, schaltet man auf die Fre-quenz f4 (10 MHz) um. Nun sortiert sich das Feld der Organoide in den Wells um, die letalen oder geschädigten schweben ganz oben und die vitalen sehr niedrig, liegen aber nicht auf dem Wellboden. So können die weniger erfolgreichen Kulturen entnommen werden, auch wenn sich mehrere Organoide in einem Well befinden. Soll nun der vitale Teil der Organoide eingefroren werden, schaltet man auf Frequenz f3 (5 MHz), bei der die vitalsten Organoide (mit Zellen niedriger Membranpermeabilität und leitfähigem Zytoplasma) bereits schwache positive DEP zeigen. Sie werden auf die Oberfläche der Boden-Elektrode gedrückt, wo sie definierter und mit schnelleren Raten abgekühlt werden können.

Eine weitere Möglichkeit mit Bedeutung für die Organoid-Kultur ist über die Wahl der Frequenz f2 (1 MHz) zu erreichen. Ein Ziel der definierten Organoid-Kultur ist es, in der Größe möglichst einheitliche Zellaggregate heranwachsen zu lassen. Wie bei f2 ersichtlich, zeigen die vitalsten Organoide bei einer Besiedlung mit 1000 Zellen gerade einen Nulldurchgang der DEP-Kräfte, d.h. liegen auf dem Boden des Wells auf, während die schlechteren und geschädigten höher schweben. Hat man also ein Zentral-Bead der in Fig. 8 beschriebenen Eigenschaften (obere Legende der Graphik) in allen Wells genutzt und eine Population vitaler Organoide, dann weiß man genau, wann sie einen Bewuchs von ca. 1000 Zellen erreicht und zum Einfrieren oder der Ernte bereit sind. Das ist mit einer Genauigkeit von etwa +/- 5% mög-lich und über die Levitationshöhenverringerung kann auch die Kinetik (Wachstumskurve) er-fasst werden.

Bisher wurde der einfachste Fall beschrieben, dielektrische Elemente dieser Art für die Organoid-Kultur und Charakterisierung passend zu gestalten. Es handelte sich um homogene Ma-terialien oder Mischungen, bisher einen Zentralkörper, der sich am Ende in dem gewachse-nen Organoid befindet. Dieses Material kann in folgender Weise aufgebaut und variiert wer-den, solange die gewünschten dielektrischen Eigenschaften auftreten (zur Kontrolle können diese vor einem Bewuchs mit Zellen über die Levitationshöhe oder eine Impedanzmessung getestet werden): Als Zentralkörper, wobei die Form von der einer Kugel abweichen kann (Ellipsoid, Zylinder, Kegel). Wie Messungen und Berechnungen zeigen, verschieben sich in Abhängigkeit von der Form die Spektren geringfügig, was bei der Auswahl berücksichtigt werden kann und es gibt Vorzugsrichtungen des Gebildes im E-Feld).

Als Beimischung kleinerer oder gleichgroßer Teile zu den Zellen, die durch das Feld lose in das Zentrum gedrückt und dort einen Zentralkörper aufgrund ihrer Dipol-Dipol-Wechselwirkungen bilden, der dann von den Zellen bewachsen wird, was permanente Feldapplikation verlangt.

Indem kein Innenkörper, sondern eine Hohlkapsel ausgebildet wird, in der die Zellen wachsen (es ist eine gängige Technik der Biotechnologie Zellaggregate zu verkapseln (z.B. mit hochgereinigten Alginaten zum Schutz der Zellen vor Immunangriffen bei Implantation)).

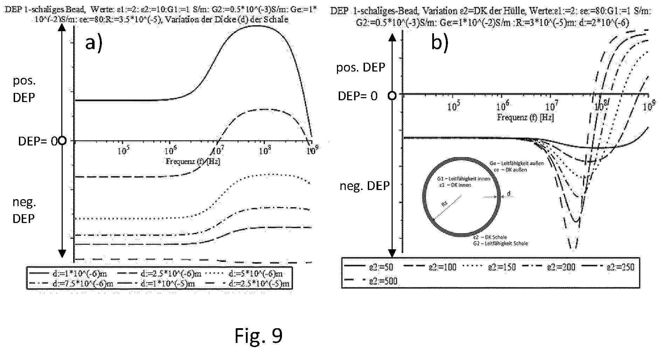

Derartige Objekte können auch aus mehr als nur einem Dielektrikum komponiert werden, woraus sich komplexere DEP-Spektren ergeben. Das wird bereits deutlich, wenn man sich ein einschaliges Bead in seinem DEP-Spektrum ohne Zellen anschaut (Fig. 9).

Fig. 9: Mit einem Zentralkörper, der aus zwei Dielektrika besteht, einem Innenraum mit einer Hülle, stehen bereits mehrere Parameter zur Verfügung, über die nach geeigneten DEP-Spektren gesucht werden kann. In a) ist gezeigt, dass die Variation der Dicke der Schale bereits eine gute Verschiebbarkeit des DEP-Spektrums zwischen positiver und negativer DEP erlaubt. Eine Variation der Dielektrizitätskonstante der Hülle ist in b) dargestellt. Solche Dielektrizitätskonstanten-Werte sind durchaus realistisch und technisch über eine Beschichtung mit z.B. Bariumtitanat oder Strontiumtitanat erreichbar.

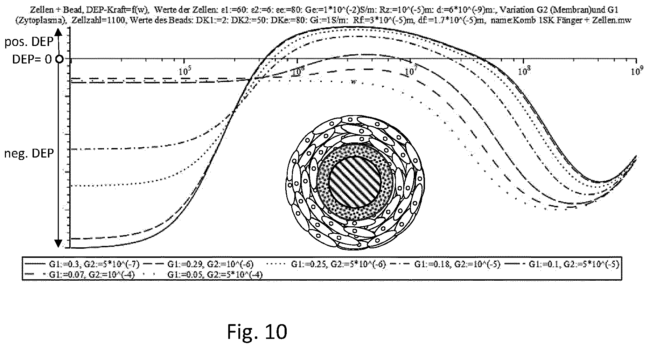

Mit komplexen dielektrischen Körpern lässt sich das DEP-Spektrum der Zellen nicht nur verschieben, sondern auch verformen (beispielhaft in Fig. 10 dargestellt). Man gewinnt breite Frequenzbereiche von mehr als einer Dekade, in denen die Zelleigenschaften (vgl. hierzu Fig. 2) weit aufgefächert sind und damit Separationen verbessert werden.

Fig. 10: Organoid mit einem Zentralkörper, der aus zwei Dielektrika besteht (ein leitfähiges inneres Dielektrikum mit geringer Dielektrizitätskonstante (schraffiert) und einer weniger leitfähigen Hülle (G-Hülle ~ 10^-3 S/m) einer Dielektrizitätskonstante von 50). Das sind realistische Werte, die technisch problemlos umgesetzt werden können. Der Komposition von zusammengesetzten Körpern, die auch aus Sektoren bestehen können, sind keine Grenzen gesetzt, erfordern aber umfangreiche Modellierungen und Testmessungen, da solche Verhältnisse nur numerisch berechenbar sind.

In der nachfolgenden Tabelle sind eine Vielzahl kommerziell erhältliche Beads mit ihren passiven elektrischen Eigenschaften aufgelistet und nach Typen (A bis E) in Bezug zur Nutzung für die Organoid-Manipulation klassifiziert.

**Tab. 1: Kommerzielles Bead-Angebot und elektrische Eigenschaften:**

| | **Bead-Material** | **Dielektrizitätskonstante** | **Leitfähigkeit** |
|---|---|---|---|
| Typ A | SiO₂ | 3,7 | <10^-20 S/m |
| Typ A | Polyurethan | 4-8 | <10^-10 S/m |
| Typ A | Al₂O₃ | 9-10 | <10^-12 S/m |
| Typ A | Polyamid | 3-4 | <10^-12 S/m |
| Typ A | PMMA | 2,8-3 | <10^-15 S/m |
| Typ C | Titanoxid | 60 - 800 | <10^-14 S/m |
| Typ A | Polyethylen | 2,4 | <10^-12 S/m (10^-8 -10^-5 S/m) |
| Typ B | Sephadex G10 (dichtestes) | 20? | Außenlösung -50 S/m? |
| Typ B | Sephadex G50 (mittleres) | 30? | Außenlösung -30 S/m? |
| Typ B | Sephadex G100 (oberes) | 50? | Außenlösung -10 S/m? |
| Typ A | Glasmikrokugeln | 2 | <10^-20 S/m |
| Typ C | Bariumtitanat | 200 - 1000 | <10^-12 S/m |
| Typ B | Alginat-Beads | 60? | Außenlösung -10 S/m? |
| Typ D | leitfähiges Polymer | 1-4 | 10^-4...10 S/m |
| Typ A | Silicon | 3,2 | <10^-14 S/m |
| Typ E | Bariumsulfat | 11,4 | 2,8*10^-6 S/m |
| Typ E | Latex | 24 | <10^-6...10^-5 |

Im angebotenen Produktspektrum ist keines der Materialien ohne Modifikation geeignet, da die gemäß einigen Ausführungsbeispielen verwendeten Parameter im bisherigen Anwendungsspektrum der kommerziellen Materialien nicht von Bedeutung sind. Materialien vom Typ A haben eine viel zu geringe Leitfähigkeit (5 bis 10 Dekaden! zu niedrig), die nur bedingt durch Zumischungen angepasst werden können (evtl. bei PMMA, Polyamid, Polyethylen und Polyurethan). Materialien Typ B haben schwer zu definierende Leitfähigkeiten, da sie einerseits porös die Außenlösung aufnehmen, andererseits wegen der dadurch großen Oberfläche mit Oberflächenladungen schwer zu berechnen sind. Typ C, Bariumtitanat auch Strontiumtitanat in Reinform eignen sich ebenfalls nicht. Auch hier muss die Leitfähigkeit angehoben werden, was wiederum die hohe Dielektrizitätskonstante absenkt, die bei diesen Materialien gerade von Vorteil ist. Materialien vom Typ D sind zumindest aus der Literatur bekannt dafür, dass man ihre Leitfähigkeit recht gut kalibrieren kann. Eine günstige Ausgangsposition bieten die Materialien nach Typ E, doch auch hier muss eine Leitfähigkeitserhöhung erfolgen. Die Zusammenstellung zeigt jedoch, dass genügend Ausgangsmaterial für Modifikationen vorliegt, so dass die gewünschten Mikroelemente definierter Form hergestellt werden können.

Bezüglich der dielektrischen Ausgangsstoffe bieten sich vor allem auch Materialien an, die in der Chirurgie und Implantationsmedizin breite Anwendung finden. Deren Vorteil besteht darin, dass sie auf biologische Verträglichkeit getestet und zertifiziert sind, zum anderen sich nach vorgebbaren Zeiten von selbst auflösen (Tage, Wochen bis Monate). Solche Materialien sind beispielhaft Monofilamente wie Polydioxanon, Poliglecapron, Polyglykonat oder Polyfilamente wie Polyglykolsäure, Cutgut und Polyglactin, die als Ausgangspunkt für die Modifikation im beschrieben Sinne ebenfalls sehr aussichtsreich sind. Hinzu kommen bioabbaubare Harze wie Poly-(3-Hydroxybuttersäure), 3-Hydrovaleriansäure-Copolymere, Chitosan, Polymilchsäure und Amid-Ester-Copolymere.

Nachdem die Brauchbarkeit dielektrischer Elemente belegt ist, sind Elektrodenkonfiguration zur Erzeugung der notwendigen Feldgradienten zu betrachten, die es erlauben: Eine möglichst gut aufgefächerte Levitationshöhe der Zell-Dielektrika-Komplexe zu erzielen, eine möglichst einfache Ansteuerung der Elektroden im Well zu finden, möglichst geringe Feldverluste und Erwärmung zu produzieren.

Hier kann z.T. auf bekannte Lösungen zurückgegriffen werden, die allerdings an die Well-Formate und die Parallelschaltung entsprechend der erfinderischen Lösung angepasst werden müssen. Die Feldanregung kann in zweierlei Weise erfolgen: als alternierendes Feld (AF, Wechselfeldansteuerung) oder als wanderndes Feld (RF, Rotationsfeldansteuerung).

Bei den hier vorgestellten Lösungen ist eine AC-Ansteuerung vorzuziehen, da in den Zuführungen nur zwei Phasen (0° und 180°) in jedes Well geführt werden müssen, während es bei rotierenden Feldern mindestens drei, in der Regel aber 4 Phasen sind (0°, 120°, 240° bzw. 0°, 90°, 180°, 270°), was entsprechend viele Zuleitungen erfordert. Der Vorteil einer AF-Ansteuerung besteht darin, dass in einem Multiwell-Format die Elektroden in einfacherer Weise (weniger Leitungszuführungen und Ebenen für die Elektroden und deren Isolation) erforderlich sind, während bei der RF-Ansteuerung zwar mehrere Ebenen mit Isolationsschichten nicht zu vermeiden sind, allerdings auch pro Phase räumlich offene Felder (kein geschlossenes Feldminimum) genutzt werden können, deren Fokussier- und Levitationseffekt erst aus den mehrfachen Umläufen des Feldes entsteht. Damit lässt sich die Zahl und damit auch die Fläche der Elektroden pro Umlauf verringern, was auch zur Verringerung der Last des Gesamtsystems der Multiwell-Platte am Generator beiträgt. In Fig. 11 sind beide Varianten und günstigen Mikroelektrodenanordnungen in den Wells über die Feldverläufe verdeutlicht.

Fig. 11: In a) bis e) ist ein RF-Anregung gezeigt, bei der jeweils nur zwei der drei planaren Elektroden angesteuert und in drei Phasen das Feld von Elektrodenpaar zu Elektrodenpaar springt (Äquipotentiallinienbilder a bis c, hier im Uhrzeigersinn, Anregung mit Rechtecksignalen). Erst ein vollständiger Umlauf führt zu einem in x-,y-Richtung geschlossenen E-Feld die (Überlagerung der 3 Phasen im Äquipotentialbild von oben gesehen ist in d) gezeigt). In e) ist der Betrag des elektrischen HF-Feldes in 3D dargestellt. Gemäß einigen Ausführungsbeispielen ordnen sich die Organoide auf der z-Achse an und wird so weit nach oben gedrückt, bis die Sedimentationskraft genau durch die neg. DEP-Kraft kompensiert wird.

f) und g) zeigt eine AF-Quadrupolansteuerung. In f) blickt man in den nun weit tieferen Feldtrichter (Betrag der Feldstärke) hinein. Ein Organoid würde wieder in einer seiner Vitalität entsprechenden Höhe auf der z-Achse schweben und kann durch die Feldbarrieren nicht in x- oder y-Richtung entweichen. In d) ist der Feldlinienverlauf am Boden mit den 4 planaren Elektroden in einer der 2-Phasen gezeigt. Die Elektroden alternieren wie angezeigt in der Polarisation entsprechend dem angelegten HF-Wechselspannungen. Diese Feldfalle ist weit kräftiger als die 3-Elektrodenanordnung, d.h. im Quadrupolfeld steigen die Organoide höher auf und werden besser zentriert und gehalten. Die Quadrate zeigen die Elektrodenfläche (von etwa 50 µm x 50 µm jeweils) mit der sie in Kontakt zur Wellflüssigkeit stehen. Zuleitungen müssen durch Isolationsschichten abgedeckt werden.

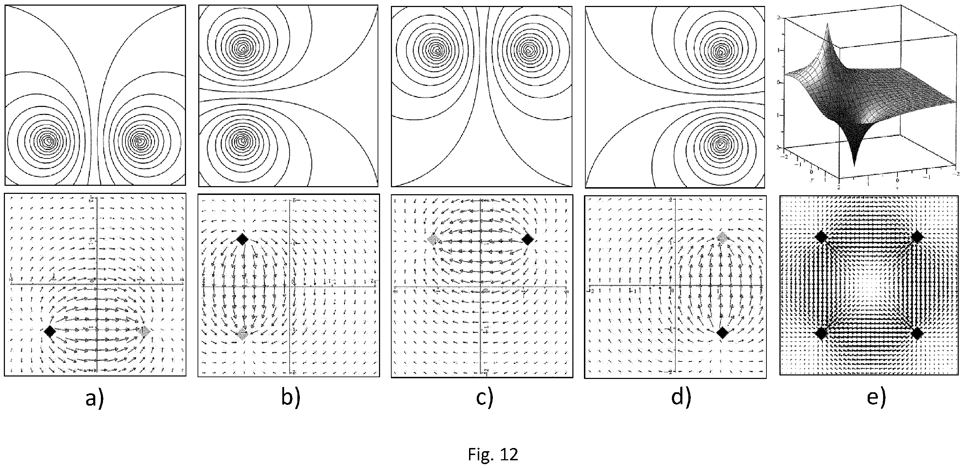

Auch Quadrupolanordnungen können in Minimalansteuerung auf jeweils nur 2 Elektroden pro Umlauf reduziert werden, wenn man das Feld umlaufen lässt, wie es in Fig. 12 gezeigt ist.

Fig. 12: a) bis d) vier Phasen der Ansteuerung der Elektroden mit einem HF-Wechselfeld (graue Viereck - negative Elektrode, schwarzes Viereck - positive Elektrode). Das Ansteuerungspärchen der Elektroden spring in der Polarität jeweils eine Elektrode im Uhrzeigersinn voran. Oben sind die jeweiligen Äquipotentiallinien, unten die E-Feldvektoren dargestellt. Die Abbildung e) zeigt oben die Potentialverteilung in einer der vier Phasen, die zu einem offenen Feld führt. In e) sind die vier Feldphasen überlagert, was zu einem geschlossenen Feldtrichter (gesehen von oben) mit dem E-Feld-Minimum im Zentrum führt.

Nachfolgend werden weitere Elektrodenanordnungen, wie sie in jedem Well umgesetzt werden können, nur noch in kompletter Ansteuerung (als geschlossener Feldverlauf) dargestellt. Jede dieser Anordnungen könnte aber auch in der in Fig. 12 gezeigten Ansteuerungsart in Phasen betrieben werden.

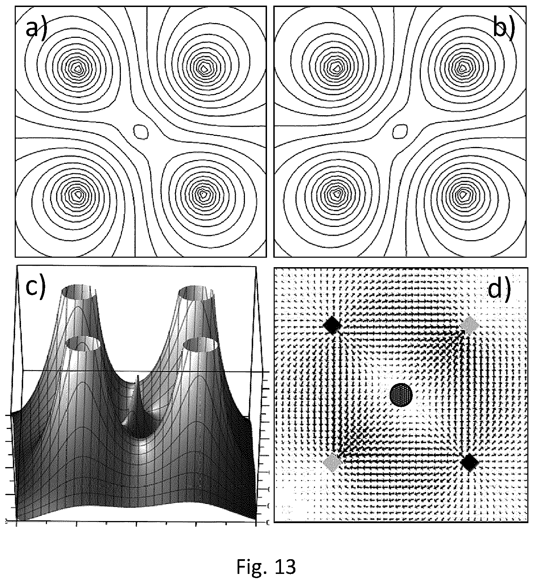

Eine weitere sehr effektive Anordnung zur Ausbildung eines Feldtrichters mit steuerbarer Levitation stellt ein Quadrupol mit Zentralelektrode dar (Fig. 13).

Fig. 13: Die planare Anordnung der Elektroden in Form des Quadrupols aus Fig. 12 ist um eine Zentralelektrode (in d) rund ausgeführt) ergänzt, die auf ein festlegbares Potential gelegt werden oder auch alternieren kann. Die Potentialverteilung, jeweils zur Halbwelle der Wechselspannung, ist in a) und b) in Form der Äquipotentiallinien von oben gesehen dargestellt. Daraus ergibt sich eine E-Feld-Verteilung (Betrag der Feldstärke) wie sie in c) abgebildet ist. Der Vorteil dieser Anordnung ist eine steuerbare z-Komponente der DEP-Kräfte, die bei kleinen Organoiden allerdings zu einem seitlichen Verdriften aus der Zentrallage führt.

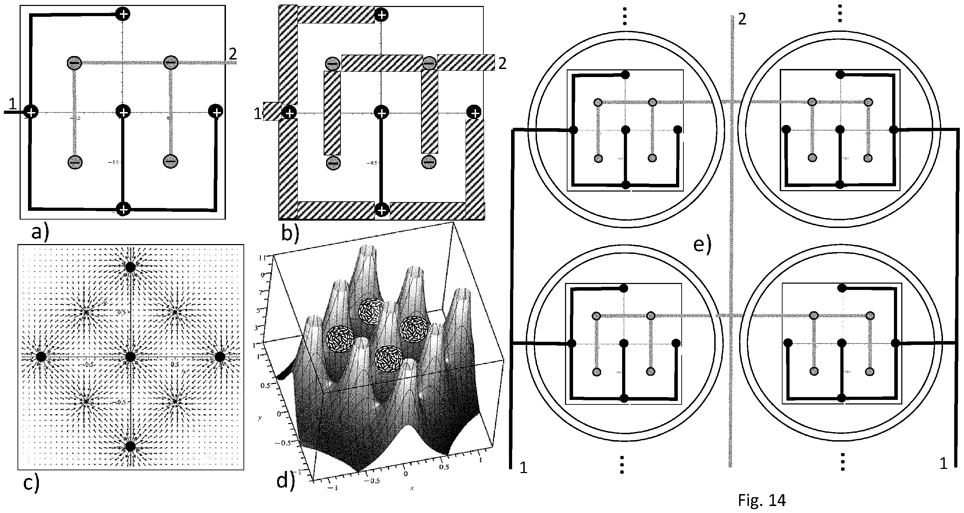

Mit zunehmender Zahl der Elektroden wird es schwieriger, Mehrebenen-Verdrahtungen zu vermeiden. Am Beispiel einer 8-Elektroden-Anordnung wird nachfolgend (Fig. 14) jedoch demonstriert, dass auch dieses Problem über eine 1-Ebenen-Verdrahtung zu lösen ist und in welcher Weise offene und isolierte Elektrodenbereiche zu gestalten sind.

Fig. 14: 8-Elektrodenanordnung in planarer Ausführung auf dem Boden von Multiwell-Platten, z.B. einem Glas- oder Plastiksubstrat, zur Demonstration, wie mehrere Organoide in einem Well in geordnete Levitation gebracht werden können. In a) ist die Anordnung der Elektroden und ihre Polarität bei Anliegen einer der Halbwellen dargestellt. Mit der nächsten Halbwelle der Wechselspannung wechselt die Polarität der Elektroden. Es werden zum Betrieb nur zwei Zuleitungen (1) und (2) benötigt. In b) sind in schraffierter Darstellung die Bereiche gezeigt, die isoliert werden müssen, da sie ansonsten die Ausbildung der vier Feldminima stören würden. Das kann eine 1 µm dicke SiO2-Schicht oder ein Lack sein, wie er in der Halbleitertechnik zur Isolierung von Bauteilen prozessiert wird. Das Feldstärkebild in der Bodenebene zeigt 4 Minima. Wie in d) dargestellt, würden 4 Organoide in quadratischer Formation schweben. Die Verknüpfung benachbarter Elektrodengruppen ist in e) am Beispiel von vier Wells gezeigt, die Reihe kann nach oben und unten fortgesetzt werden.

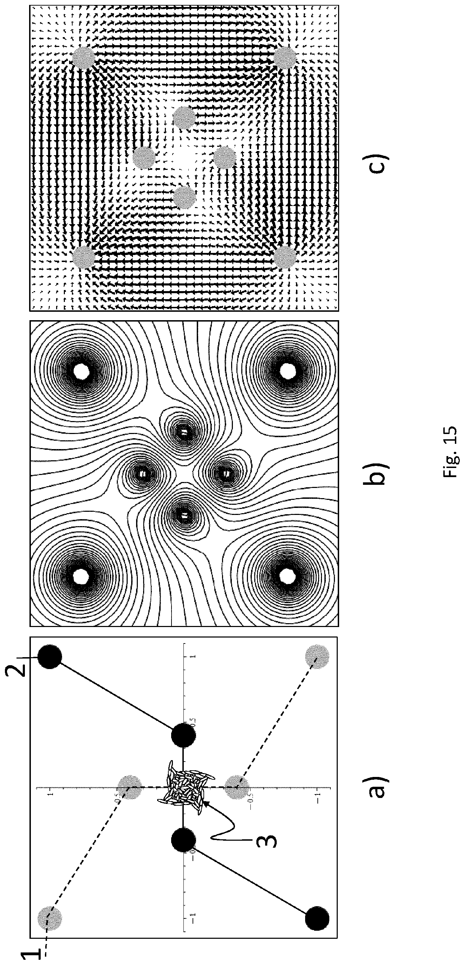

Beispielhaft für die Möglichkeiten zur Kultur von Organoiden ist in Fig. 15 eine 8-Elektrodenanordnung (innerer und äußerer Quadrupol) dargestellt (a), durch die Verdrehung um 45 ° entstehen auch leicht deformierte Feldminima (c), so dass sich wachsende Organoide nicht sphärisch, sondern kissenförmig ausbilden würden. Gibt man später weitere Zellen oder eine andere Art zu, würden diese sich als Schicht auf der Oberfläche ansiedeln und diese bedeckend wachsen. Die Außenelektroden stabilisieren die Halterung im Inneren des Wells, die inneren vier Elektroden sorgen für die Levitation.

Fig. 15: Doppel-Quadrupolanordnung im HF-Wechselspannungsmodus. An den Verbindungslinien in a) zwischen den Elektroden (schwarz und grau), die zum Generator führen, ist auch hier eine 1-Ebenen-Lösung mit Isolierung gegeben. Sowohl im Äquipotentialbild b), als auch dem Verlauf der elektrischen Feldstärke ist, der Einfluss äußerer Elektroden erkennbar, die zu anderen als sphärischen Organoiden (3 in a)) führt. Gemäß einigen Ausführungsbeispielen kann über derartige Kombinationen und Anordnungen von mehr als vier Elektroden die Form der Zellaggregate und Organoide beeinflusst und bestimmt werden.

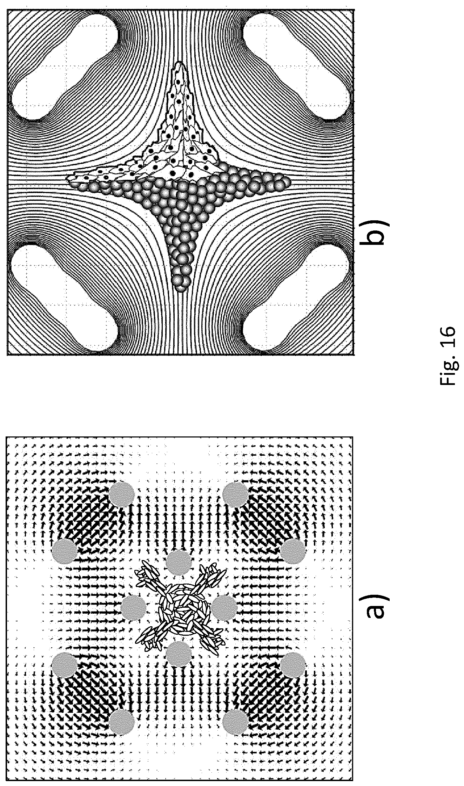

Zwei weitere Beispiele für Feldverläufe die die Geometrie der Zellaggregate vorgeben, sind in Fig. 16 ausgeführt.

Fig. 16: Elektrodenanordnungen und Feldlinien- bzw. Potential-Verläufe zu Beeinflussung der Geometrie der Organoide. In a) handelt es sich um kräftige äußere Felder und einen schwächeren Quadrupol innen, in b) um eine Quadrupol-Anordnung alternierender Polarität mit balkenförmigen, schräg angeordneten Elektroden. Dielektrische Beads würden sich in sternform anordnen und würden nachfolgend in dieser Form von Zellen überwachsen.

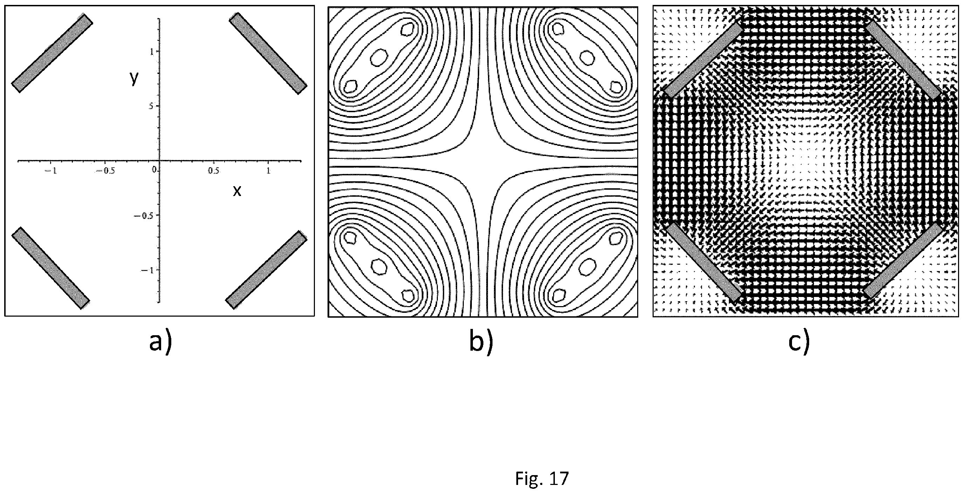

Die Positionierung der Organoide muss in alle drei Raumrichtungen in möglichst ausgeglichener und kräftiger Form erfolgen, damit sie auch weit oben schwebend stabil auf der Zentralachse gehalten werden. Über die Art und Größe der Elektrodenflächen, wie sie in zwei Beispielen in Fig. 17 gezeigt sind, lässt sich die laterale Fokussierung (x- und y-Richtung) verstärken. Dabei muss in den Wells der Multiwell-Platten allerdings ein Kompromiss eingegangen werden, denn je größer die Elektrodenfläche gestaltet wird, umso geringer wird der Gesamtwiderstand der Anordnung, weil eine größere Elektrodenfläche zu einem höheren Stromfluss durch die leitfähige Zellkulturlösung führt (siehe hierzu weiter hinten - Gesamtwiderstand von Multiwell-Platten).

Fig. 17: Längliche Elektroden (grau) verstärken die Fokussierung dielektrischer Objekte auf die Mittelachse deutlich. Über die Elektrodengeometrie kann Einfluss auf die x-,y- und z-Ausprägung der Feldgradienten genommen werden.

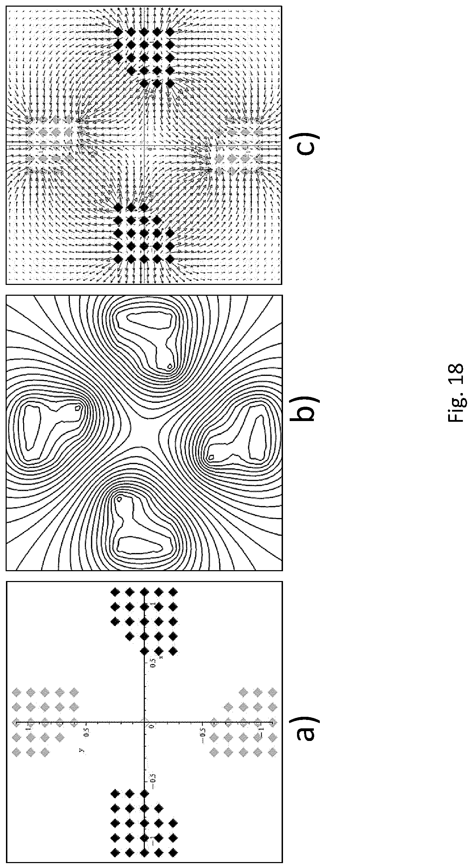

Feldbeeinflussungen lassen sich aber auch erreichen, ohne dass die Stromflüsse deutlich ansteigen. In Fig. 18 sind irreguläre Elektroden gezeigt, die nicht aus einer zusammenhängenden Fläche bestehen, sondern so isoliert wurden, dass kleine Oberflächenquadrate frei bleiben, d.h. nur diese stehen in direktem Kontakt mit der Zellkulturlösung und liegen alle auf dem gleichen Potential pro Phase. In den Wells ist diese eine weitere Ausführungsvariante, die DEP-Feldkräfte zu formen und Feldverluste zu verringern.

Fig. 18: Quadrupol-Elektroden-Konfiguration, wobei die Elektroden aus jeweils 22 kleinen, quadratischen Flächen geformt werden (a) und dadurch zu veränderten Feldverteilungen führen (b, c). Da die Elektrodenzuführungen ohnehin abgedeckt und isoliert werden, was in der Regel über Masken, Besputtern oder auch im Siebdruck erfolgt, lassen sich auf großflächigen Elektroden feine Muster und beliebige Elektrodengeometrien und damit Feldverläufe erzielen.

Gemäß einigen Ausführungsbeispielen ist dieser Art der Elektrodengestaltung in den Wells der Vorzug zu geben, da auf sehr einfache Weise komplizierte Elektroden erzeugt werden können, bei gleichzeitiger Begrenzung der Elektrodenfläche.

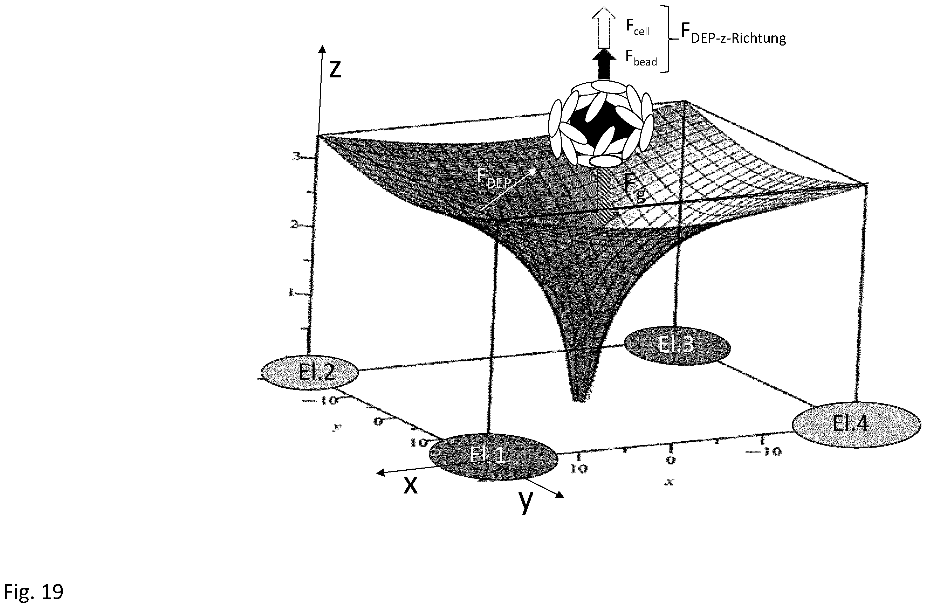

Bisher wurden ausschließlich planare Elektrodenanordnung erläutert. In denen fallen die 3D-Feldtrichter relativ flach aus, d.h. werden schnell mit der Höhe breiter. Das bedeutet, dass die Organoide vergleichsweise niedrig schweben. Gemäß einigen Ausführungsbeispielen der Abmessungen sind Abstände zwischen den Elektroden zwischen 200 und 800 µm. Die Levitationshöhe der Organoide liegt in etwa der gleichen Größenordnung, etwas niedriger Fig. 19.

Fig. 19: Veranschaulicht ist der DEP-Kraft-Trichter den 4 planare Elektroden am Wellboden erzeugen. Die Levitationshöhe des Organoiden wird durch die Kompensation zwischen Sedimentationskraft (Fg) und der Überlagerung der DEP-Kräfte von Bead und Zellen (Fcell, FBead) bestimmt. Die Aufreihung folgt in etwa dem Kurvenverlauf am Trichterrand.

Eine stärkere Levitation und Fokussierung in Richtung der z-Achse (Mittelachse des Wells) lässt sich über eine Anordnung von Elektroden am Wellboden in verschiedener z-Höhe erreichen, wie sie in Fig. 20 abgebildet ist, die sich in den Well-Formaten, die am Boden konisch zulaufen leicht verwirklichen lässt. Hier befindet sich eine Ringelektrode etwas oberhalb der Bodenelektrode.

Fig. 20: Gemäß einigen Ausführungsbeispielen eine Anordnung und Feldverteilung in einem 2-Elektrodensystem am konisch zulaufenden Wellboden von Multiwell-Platten, bestehend aus einer Ringelektrode und einer Zentralelektrode am Boden des Wells (die schwarze Wellwand ist hier schematisch dargestellt). In a) ist ein Wellboden einer 384er-Wellplatte im Schnitt mit der Anordnung zweier Elektroden abgebildet. Eine Elektrode befindet sich zentral am Wellboden eine zweite als Ring-Elektrode im Abstand z (vgl. b) darüber. Typische Abstände für x und z sind 200 bis 800 µm. Die räumliche Anordnung führt zu einer FeldstärkeVerteilung, wie sie in b) gezeigt ist. Wie in Fig. 18 eingeführt und in a) dargestellt ist es zweckmäßig die Ringelektrode alternierend abzudecken, um die Wärmeentwicklung und Verluste zu minimieren. Es bildet sich ein Feldminimum weitab der Elektroden in der Lösung heraus (b und c). In c) sind die Äquipotentiallinien im z-x-Schnitt durch das Zentrum des Wells aufgetragen. Legt man entsprechend der Beschreibung in Fig. 8 die Frequenz f1 (ca. 60 kHz) an so wird ein vitaler Organoid im Feld ganz nach oben gedrückt, soll dieser, nachdem er über seine Levitationshöhe charakterisiert wurde, eingefroren werden, schaltet man auf die Frequenz f2 oder f3 (ca. 1 bis 5 MHz) um und kühlt das Well von außen und unten. Durch die nun pos. DEP-Kräfte wird das Organoid auf den Boden gezogen, wo eine definierte Kühlung bis zum Einfrieren erfolgt. Beim Auftauen wird dann wieder f1 angelegt und das Organoid steigt in die seinen Kryoveränderungen entsprechende Levitationshöhe auf. Wie bereits erläutert, verhalten sich geschädigte oder letale Organoide invers, können leicht erkannt und vor dem Einfrieren von oben entnommen werden.

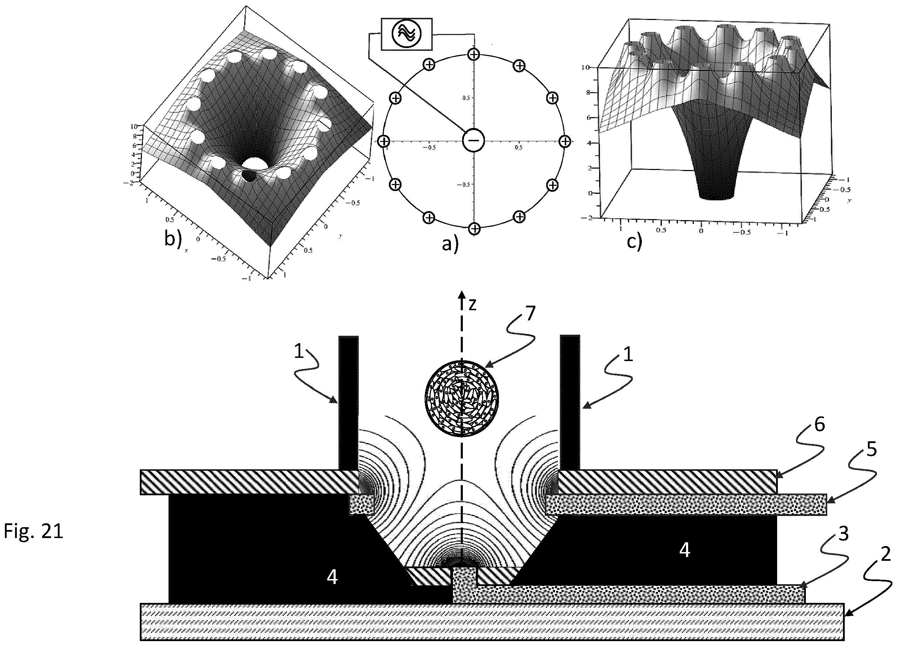

In Fig. 21 ist die vorteilhafte Zentral-Ring-Elektrodenkonfiguration in einer planaren Ausführung dargestellt, wie sie in Schichttechnik auf der Bodenplatte der Wells realisiert werden kann.

Fig. 21: In a) ist die vorteilhafte Konfiguration aus einer Ring- und einer Zentral-Elektrode gezeigt, in b) und c) die Potentialverteilung des Feldes bei Abdeckung des Rings in 12 Segmenten mit einer Isolationsschicht. Darunter ein Schnittbild durch ein Well mit der Wellwand (1) und der Bodenplatte (2). Auf der Bodenplatte 2 ist die Zuführung und der Zentralteil der Boden-Elektrode (3) in planarer Schichttechnik prozessiert worden, darüber eine Spacerschicht (4) mit der der Abstand z zur Ringelektrode (5) einstellbar ist. Die Zuführung der Ring-Elektrode ist auf dem Spacer wieder in Planartechnik erfolgt. Eine weitere Isolationsschicht (6) bedeckt die Teile der Ring-Elektrode und Zuführung, die nicht mit der Flüssigkeit im Well in direkte Verbindung kommen soll. Die Ausbildung der Äquipotentiallinien im Wellbereich ist als Linien dargestellt und ein schwebender Organoid (7) auf der Mittelachse des Wells.

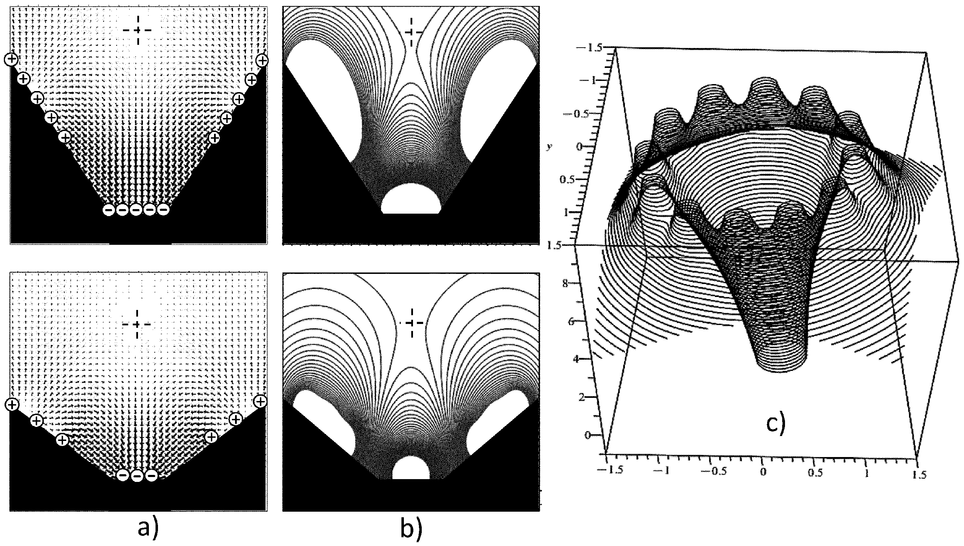

Der Einfluss des konischen Verlaufs und auch der Elektrodenfläche auf die Ausbildung des DEP-Krafttrichters ist in Fig. 22 dargestellt. Über die (je spitzer) lässt sich das Feldminimum weit nach oben schieben. Da diese Form in gewissen Grenzen in den Multiwell-Platten variiert werden kann, liefern die Bilder in Fig. 22 realistische Winkel.

Fig. 22 Unter a) ist die Feldstärkeverteilung für zwei konische Ausführungen des Wellbodens dargestellt, in b) die dazugehörigen Äquipotentiallinien, wieder einer segmentierten aber nun breiter ausgeführten Ringelektrode und einer Zentralelektrode. Aus c) wird deutlich, dass sich über eine 3-dimensionale Elektrodenanordnung sehr steile Levitationstrichter erzeugen lassen, die die Nutzung für die Kryocharakterisierung gemäß einigen Ausführungsbeispielen weiter verbessern.

Durch Einführen eines weiteren Elektrodenringes oder vier schräge Elektroden lassen sich die Feldgradienten in Richtung der z-Achse in gewissem Umfang linearisieren und verlängern, bei gleichzeitig guter Zentrierung. Zwei beispielhafte Ausführungen sind in Fig. 23 berechnet.

Fig. 23: 2-Ring-Elektroden-Zentral-Elektrodensystem im Wellboden eines 384er Wells (der Boden muss nicht konisch auslaufen, da die Zentral-Elektrode mit dem 1. Ring ein Feld ausbilden, das eine Zentrierung der Organoide erzwingt). In a) die Elektrodenanordnung, in b) die Äquipotentiallinien im Schnittbild und in c) die Verteilung der Feldvektoren. In d) und e) ist ein weitgehend linearisierter Feldtrichter in den Äquipotentiallinien (links) und der Feldverteilung (rechts) in mehreren Ebenen gezeigt. Es handelt sich im Grunde um eine Quadrupolanordnung (2) mit Wechselspannungsanregung (Phase 1 -schwarz, Phase 2 - grau). Die Elektroden laufen an der Tubewand (1) schräg nach oben, wodurch sich die gewünschte Feldverteilung ergibt. Wie in e) gezeigt, kann die Kontaktierung am Boden erfolgen, alternativ kann ein Elektrodeneinsatz auch von oben in die Wells eingesetzt werden, so dass die Wechselspannungsversorgung von oben erfolgt.

Gemäß einigen Ausführungsbeispielen ist nun auch die Dauerfeldapplikation über Stunden und Tage und permanentes Schweben von Organoiden möglich. Bisher war das entsprechend der DEP-Zellspektren (vgl. auch Fig. 2) nur im Bereich der neg. DEP möglich. Die tritt aber nur im kHz-Bereich auf, in dem gleichzeitig die Zellhüllmembranen maximal polarisiert und ein zusätzliches, dem zelleigenen in der Amplitude vergleichbaren Transmembranpotential in der Frequenz des Anregungsfeldes induziert wird. Da der zusätzliche Betrag des induzierten Transmembranpotential je nach Anregungsamplitude des HF-Signals Werte bis zum dielektrischen Durchbruch der Hüllmembran gesteigert werden kann, wird deutlich, dass diese Belastung der Zellen unerwünscht ist und eine Dauerbefeldung (Stunden und Tage, Wochen) bisher ausschloss.

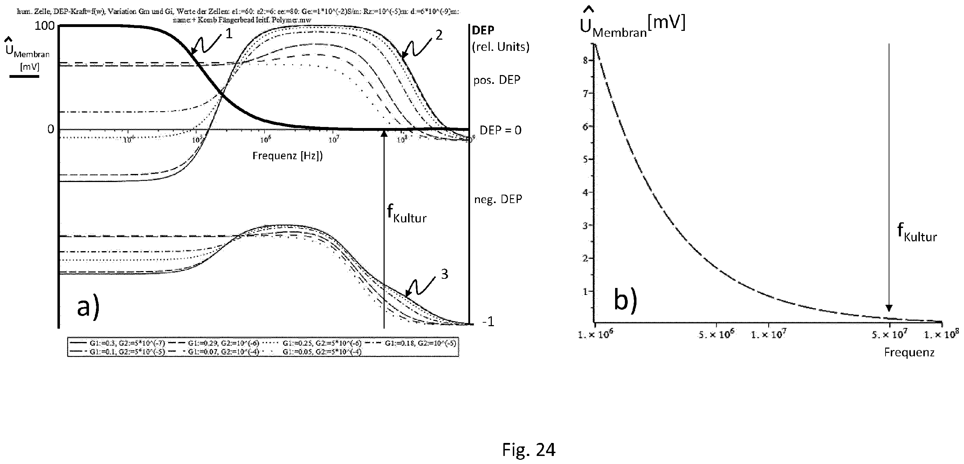

Fig. 24: Kultivierung von Zellaggregaten und Organoiden bei Langzeit-Feldapplikation (hier beispielhaft bei einer Frequenz (fKultur) von 50 MHz. In der Graphik a) ist der Verlauf des induzierten Peak-Membranpotentials (dicke Kurve (1), linke Ordinate) und der Verlauf der DEP-Spektren vitaler bis geschädigter Zellen analog zu Fig. 2 in relativen Einheiten (rechte Ordinate) dargestellt. Bei Feldapplikation mit Amplituden von über 15 VSS werden alternierende Potentiale von bis zu 100 mV über die Membran induziert. Das entspricht der Größenordnung des natürlichen Membranpotentials und stellt eine nicht unerhebliche Belastung der Zellen dar. Hinzu kommt, dass auch nur für einen Teil der vitalen Zellen (mit Membranleitfähigkeiten < 5*10-6 S/m, Kurvenschar (2)) in einem Frequenzbereich von 103 bis 105 Hz negative DEP auftritt. Wegen der hohen Belastung durch das induzierte Transmembranpotential (UMembran) war bisher eine Langzeit-Feldapplikation mit Wachstum von tierischen und humanen Zellen im levitierten Zustand nicht erfolgreich. Wachsen die Zellen nun auf einem Bead (in a) beispielhaft mit der Leitfähigkeit Gi= 5*10-3 S/m, einer DKi=4 und einem Radius von 35 µm), so tritt bei einem Bewuchs mit 300 Zellen (mehrschichtiger Organoid) die Kurvenschar (3) auf. Das Zellspektrum als Funktion der Zytoplasma und MembranEigenschaften (Kurvenschar (2)) wird nicht nur weit in den neg. DEP-Bereich verschoben, sondern auch im Kurvenverlauf verändert. Der bisher für die Levitation im E-Feld wegen der pos. DEP nicht nutzbare Frequenzbereich > 105 Hz ist nun weit in den neg. DEP-Bereich verschoben. Zur Kultivierung von Organoiden in levitiertem Zustand sollte das induzierte Membranpotential so gering wie möglich gehalten werden. Wie die Graphik in b) in höherer Auflösung belegt, sinkt das induzierte Peak-Membranpotential oberhalb von 10 MHz unter 1 mV und fällt bei 50 MHz und hohen Feldstärken, wie sie üblicherweise nicht angelegt werden, auf einen Wert von 0,2 mV. Das ist etwa 1/200 des natürlichen Membranpotentials. Daher ist bei Langzeit-Feldapplikation eine Kultur- und Levitations-Frequenz ((fKultur) um 50 MHz angeraten. Daraus folgt die Frage, wenn bei dieser Frequenz nicht an der Membran, wo treten dann die Polarisationsladungen und Kräfte auf, die zum Schweben des Zell-Bead-Systems führen? Bei diesen hohen Frequenzen handelt es sich um Polarisationsladungen an den Grenzflächen des Beads und im Zytoplasma der Zelle, sowie Ladungsansammlungen in der Außenlösung an der Organoid-Oberfläche, d.h., ein nicht unwesentlicher Teil der Kräfte wirkt auf das Bead und belastet damit die Zellen nicht. Die Polarisation des Zellinneren führt durch die Ladungsbewegung lediglich zu einer geringfügigen Erwärmung, die sich als nicht schädlich erweist und über Thermostatierung der Multiwell-Platten in gewissen Grenzen kompensiert werden kann. Die Erwärmung selbst kann nicht verhindert werden, ist aber erst bei hohen Feldstärken relevant.

Die Langzeitkultivierung unter Feldeinfluss erlaubt nun auch die intensivere und nachhaltigere Formung von Organoiden, wie sie in Fig. 15 und 16 behandelt wurde. Je länger das Feld Kräfte auf die Zellen ausübt, umso stärker binden und wachsen die Zellen in dieser geometrischen Form. Des Weiteren lagern sich später zugegebene Zellen in einem Medium, in dem sich ein Organoid befindet, nicht gleichmäßig auf der Oberfläche, sondern dort an, wo das E-Feld schwächer ist, bzw. die Dipol-Dipol-Wechselwirkungen zwischen den Zellen keine parallele, sondern eine Reihenanordnung befördern. Ebenso funktioniert das bereits beschriebene Absinken der Organoide bei Zunahme der Zellzahl bis zu einem Durchmesser, bei dem sie mit ihrer pos. DEP die Wirkung des Beads (neg. DEP) übertreffen.

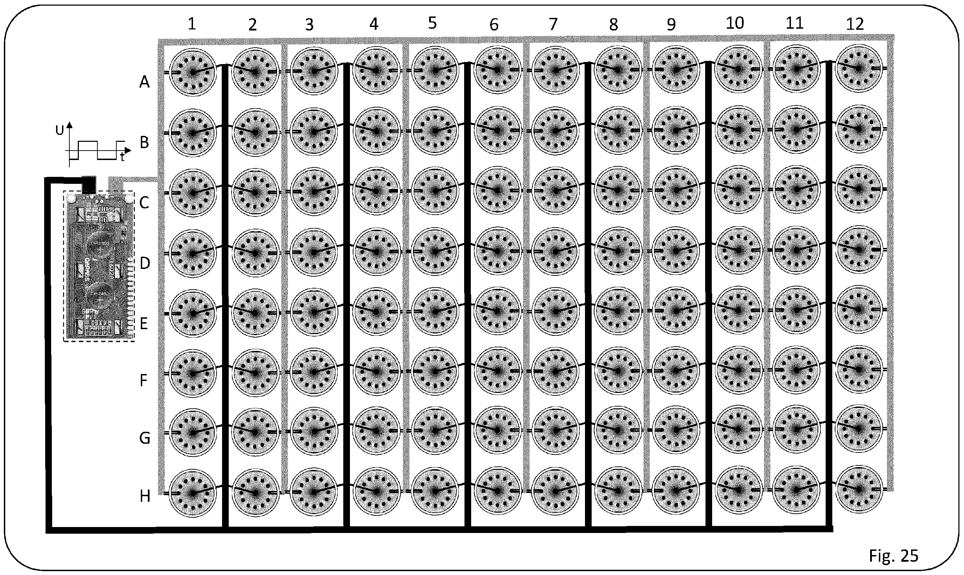

In Fig. 25 ist schematisch die Anordnung des Ring-Zentral-Elektroden-Systems für ein 96-Well-Format dargestellt. Der Generator ist hier schematisch als Leiterplatte links auf der Multiwellplatte gezeigt, das Signal kann aber auch über Kontakt-Pads eingespeist werden. Die Bezeichnung der Wells erfolgte den handelsüblichen Standards entsprechend in Zahlen und Buchstaben. Da nur 2 Elektroden pro Well kontaktiert werden müssen, kann die Elektrodenzuführung in einer Ebene, ohne Zuleitungskreuzungen wie dargestellt ausgeführt werden (z.B. auf der dünnen Plexiglasbodenplatte des Well-Systems. Die Zuführungen sind zweckmäßigerweise mit einer dünnen Isolierschicht abgedeckt und nur dort frei, wo sie im Well das E-Feld erzeugen sollen. Als HF-Signale können alle Formen, Sinus, Dreieck, Rechteck oder andere appliziert werden. Rechteckimpulse bieten sich aus folgenden Gründen als die effektivste Signalform an: sie sind bis zu GHz-Frequenzen digital erzeugbar; es stehen integrierte Schaltkreise mit kurzschlussfesten Ausgängen hoher Leistung zur Verfügung; die umgesetzte Leistung im Well entspricht der Fläche unter der Signalkurve, die beim Rechteck größer ausfällt im Vergleich zur Sinus- und Dreiecks-Anregung.

Die üblichen Amplituden liegen im Bereich von wenigen Volt, können aber kurzzeitig auf deutlich höhere Werte angehoben werden (20 V und mehr), insbesondere beim Auftauen nach der Kryokonservierung, um eine Wärmeproduktion im Inneren der Zellen zu erreichen, mit der die kritische letzte Auftauphase beschleunigt werden kann.

Fig. 25: 96-Multiwell-Platte mit Rechteckgenerator und Verdrahtung der Elektrodensysteme in einer Ebene. Die beiden Zuleitungen der HF-Wechselspannung sind zur Unterscheidung grau (für die Ringelektroden) und schwarz (für die Zentral-Elektrode) dargestellt.

Eine direkte und parallele Ansteuerung aller Wells ist nur anwendbar, wenn Zellen in wenig leitfähigen Lösungen (< 10^-3 S/m) kultiviert oder vermessen werden. Das trifft nur auf wenige Zellarten (z.B. Algen, einige Bakterien), nicht aber für tierische und humane Zellen zu. Der Elektrode zu Elektrode-Widerstand durch die Flüssigkeit des Wells entlang der Feldlinien liegt bei tierischen und humanen Zellen aufgrund der hohen Leitfähigkeit der Zellkulturmedien im unteren kOhm-Bereich, ein realistischer Wert ist 2,4 kOhm. Eine Parallelschaltung aller 96 Wells, wie in Fig. 25 ausgeführt, entspricht einer Parallelschaltung von 96 Widerständen von jeweils 2,4 kOhm, was zu einem Gesamtwiderstand von 25 Ohm führt. Das lie-ße sich generatorseitig gerade noch umsetzen, bei einem 384er Wellformat würden die resultierenden 6,25 Ohm jedoch einem Kurzschluss nahekommen.

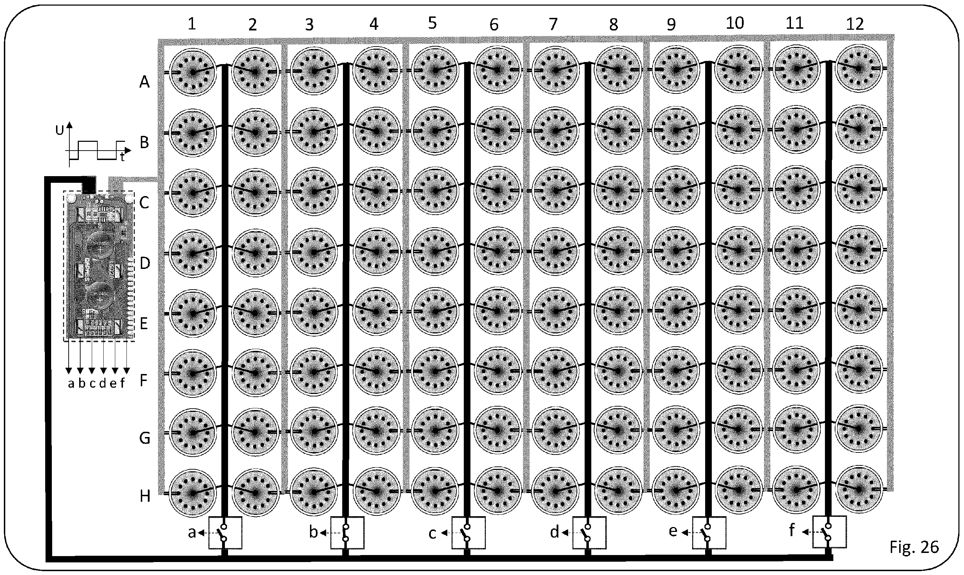

Gemäß einigen Ausführungsbeispielen ist eine temporäre Feldapplikation an einer reduzierten Zahl von Wells eine leicht umzusetzende Lösung des Problems. Zwei der möglichen Varianten sind beispielhaft in Fig. 26 und 27 dargestellt.

Fig. 26: Über elektronische Schalter (in der Regel zwei Feldeffekttransistoren) lassen sich über die Schalter a, b, c, d, e und f jeweils Gruppen von Wells an- und abschalten (pro Schalter 2 Reihen = 16 Wells). Damit liegt der Lastwiderstand am Generator bei unkritischen 300 Ohm.

Fig. 27: Durch Einfügen weiterer elektronischer Schalter (g, h, i, j, k) kann das auf jeweils eine Spalte (acht Wells) reduziert werden. Falls erforderlich, können die Wells durch Einfügen weiterer Schalter bis hinunter zur Einzelwellaktivierung verschaltet werden.

Ein alternativer Weg zur Lösung des Problems, die getrennte Einspeisung über mehrere Generatoren, Fig. 28. Diese müssen nicht zwingend auf der Well-Platte angebracht werden, sondern können auch über Kontakt-Pads eingekoppelt werden.

Fig. 28: Beispielhafte zweifach-Einkopplung von Generatorsignalen, um die gesamte Well-Platte zu befelden. Auch das lässt sich über eine 1-Ebenen-Zuleitungsführung realisieren, da nur eine der Wechselspannungszuleitung aufgetrennt werden muss (hier die schwarze Zuleitung), während der andere Pol als Masse behandelt werden kann.

Zur vollständigen Beschreibung der Erfindung fehlen nun noch die prinzipielle Umsetzung der Höhenmessung an schwebenden Organoiden und eine Darstellung des Gesamtsystems zur Kryokonservierung und Kryocharakterisierung von Zellaggregaten in Multiwell-Formaten.

In Fig. 29 ist die bereits beschriebene Autofokus-Lösung als Applikation des Standes der Technik schematisch abgebildet.

Fig. 29: Autofokus-Systeme, bestehend aus Objektiv und Kamera, die automatisch Objekte scharfstellen, können auf x-y-Tischen ohne Probleme und rasch im Wellformat von Wellmittelpunkt zu Wellmittelpunkt programmiert verfahren werden. Die E-Feldwirkung hält das Organoid exakt auf der Zentralachse (bisherige z-Richtung). Messungen ergaben, die Position wird mit einer Genauigkeit im µm-Bereich (etwa 1/200 bis 1/400 des Organoid-Durchmessers) gehalten. Das ermöglicht eine sehr gute Scharfstellung im Autofokus-Modus. Die Regelstrecke delta-x ist proportional oder auch identisch zu delta-h der Position des Organoids und wird am Autofokussystem in ein elektrisches Signal umgewandelt. In a) ist das Mikroskopbild eines Organoids im Scharfbereich, in c) außerhalb des Fokus gezeigt.

Gemäß einigen Ausführungsbeispielen kann die Höhenerfassung in diesem System auch so erfolgen, dass die Mikroskop-Einstellung konstant bleibt und die Amplitude der applizierten HF-Signale so lange verändert wird, bis das Organoid wieder im Scharfbereich des Objektivs steht. Der zur Levitationshöhe proportionale Parameter ist dann die erforderliche Amplitudenänderung der Generatorsignale.

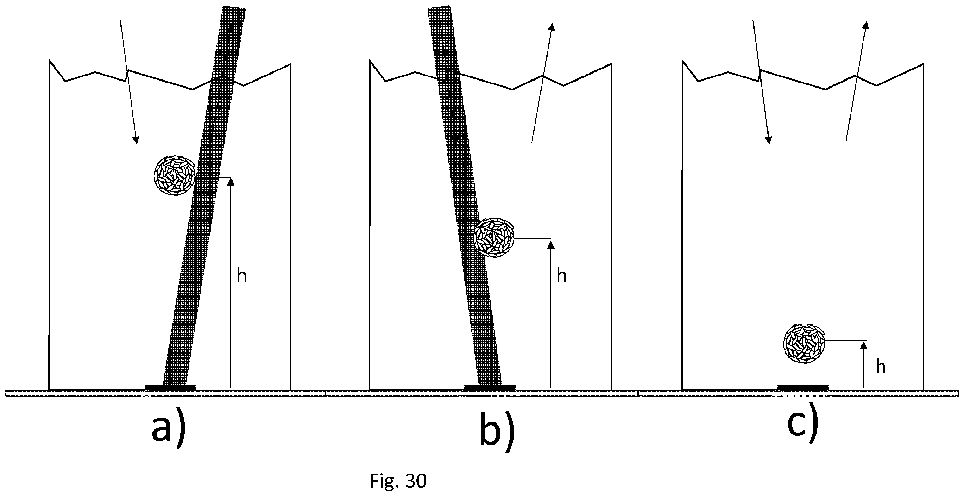

In Fig. 30 ist eine alternative Messmethode über einen eingekoppelten und am Boden (z.B. der Zentral-Elektrode) reflektierten Messtrahl dargestellt. Die Höhe h kann aus der Schwächung des reflektierten Strahls bestimmt werden.

Fig. 30: Veranschaulichung der Messung der Levitationshöhe h eines Organoids über einen schräg eingestrahlten Messstrahl, die belegt, dass es eine Vielzahl von Möglichkeiten gibt, die Position der Organoide zu bestimmen.

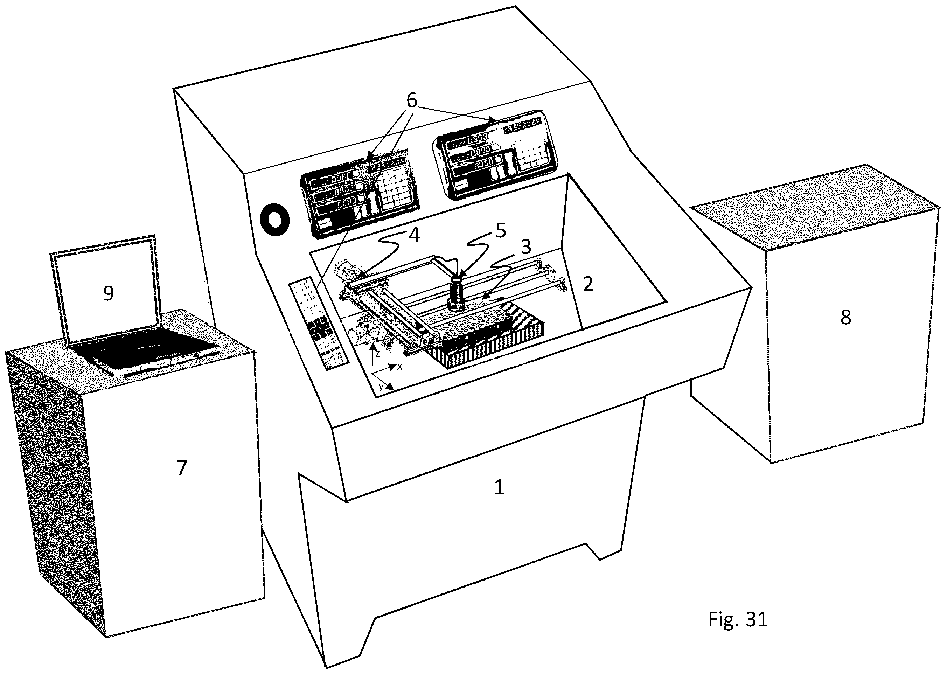

In Fig. 31 ist abschließend ein Gesamtsystem zur Kultivierung, Vermessung und Kryokonservierung von Zellkulturen in Multiwell-Formaten dargestellt. Es ist eine Kombination aus Inkubator zur Zellkultur, mit Gasversorgung (CO2-Einstellung) und Temperierung, Haube und Kryostat für die Kryokonservierung auf Temperaturen unterhalb -120 °C.

Fig. 31: Gesamtsystem zur Kultivierung und Kryokonservierung von Zellaggregaten und Organoiden in Multiwell-Plates. 1 bezeichnet das Zentralgerät mit einem einsehbaren aber hermetisch abgeschlossenen, öffenbaren Inkubator-, Mess- und Kryo-Raum 2. In dem befindet sich ein x-y-Tisch 4, mit dem das Kamera-Autofokus-System 5 von Well zu Well bewegt werden kann. Die Wellplatte 3 befindet sich auf einem Kühltisch (schraffiert). Das System kann sowohl als Inkubator (versorgt durch das Aggregat 7, als Kryocharakterisierungssystem und zum Einfrieren/Auftauen (Kryostat 8) genutzt werden. Dafür stehen die Bedieneinheiten 6 zur Verfügung. Die Datenaufnahme und Steuerung erfolgen computergestützt 9.

Das oben beschriebene Beispiel dient in keiner Weise einer Beschränkung der Erfindung. Vielmehr kann die Erfindung in vielfältiger Weise abgewandelt werden. Alle oben beschriebenen Merkmale der Erfindung können allein oder in Kombination miteinander wesentlich für die Erfindung sein.

## Patentansprüche

1. System zum Charakterisieren, Separieren, Vermehren und/oder Kryokonservieren mindestens einer biologischen Zelle, umfassend mindestens eine Vorrichtung aus mindestens einer biologischen Zelle und einem dielektrischen Trägerkörper, mindestens einen Behälter mit einem offenen Mündungsbereich und einem Bodenbereich, wobei die Vorrichtung in dem Behälter angeordnet ist, wobei der Bodenbereich mindestens eine erste Elektrode und mindestens eine zweite Elektrode zum Erzeugen eines elektrischen Wechselfelds innerhalb des Behälters aufweist, **dadurch gekennzeichnet, dass** der dielektrische Trägerkörper in einem elektrischen Wechselfeld mit einer ersten Wechselfrequenz eine schwächere negative dielektrophoretische Krafteinwirkung aufweist als in einem elektrischen Wechselfeld mit einer zweiten Wechselfrequenz, die höher als die erste Wechselfrequenz ist, und zumindest in dem elektrischen Wechselfeld mit der zweiten Wechselfrequenz eine stärkere negative dielektrophoretische Krafteinwirkung als ein Absolutwert einer dielektrophoretischen Krafteinwirkung einer einzigen biologischen Zelle in dem elektrischen Wechselfeld mit der zweiten Wechselfrequenz aufweist.

2. System nach Anspruch 1, **dadurch gekennzeichnet, dass** die erste Elektrode und/oder die zweite Elektrode als eine Ringelektrode und/oder als eine Punktelektrode ausgebildet ist, und/oder
dass das die erste Elektrode und die zweite Elektrode in unterschiedlichen Abständen zu dem Mündungsbereich angeordnet sind.

3. System nach einem der vorangegangenen Ansprüche, **dadurch gekennzeichnet, dass** das System mindestens zwei erste Elektroden und mindestens zwei zweite Elektroden aufweist, wobei die mindestens zwei ersten Elektroden vorzugsweise abwechselnd ansteuerbar sind und/oder dass die mindestens zwei zweiten Elektroden vorzugsweise abwechselnd ansteuerbar sind, wobei vorzugsweise eine der mindestens zwei ersten Elektroden und eine der mindestens zwei zweiten Elektroden gleichzeitig ansteuerbar sind, wobei der Bodenbereich vorzugsweise mindestens eine dritte Elektrode aufweist, die im Zentrum einer Quadrupolanordnung aus den mindesten zwei ersten Elektroden und den mindestens zwei zweiten Elektroden angeordnet ist.

4. System nach einem der vorangegangenen Ansprüche, **dadurch gekennzeichnet, dass** die mindestens eine erste Elektrode und/oder die mindestens eine zweite Elektrode einzeln ansteuerbar ist.

5. System nach einem der vorangegangenen Ansprüche, **dadurch gekennzeichnet, dass** die erste und zweite Elektrode Teil mindestens einer Quadrupolanordnung und/oder Oktupolanordnung von Elektroden in dem Behälter sind, wobei die erste und zweite Elektrode vorzugsweise Teil einer Anordnung mit einem äußeren Quadrupol und einem inneren Quadrupol in dem Behälter sind, wobei der äußere Quadrupol vorzugsweise zum Erzeugen eines elektrischen Wechselfelds mit einer ersten Feldstärke und der innere Quadrupol zum Erzeugen eines elektrischen Wechselfelds mit einer zweiten Feldstärke ausgebildet sind, wobei sich die erste Feldstärke und die zweite Feldstärke unterscheiden, wobei der innere Quadrupol und der äußere Quadrupol vorzugsweise um eine Mittelachse angeordnet sind und, vorzugsweise um 45°, unterschiedliche Drehwinkel zur Mittelachse aufweisen.

6. System nach einem der vorangegangenen Ansprüche, **dadurch gekennzeichnet,**
**dass** die erste Elektrode und/oder zweite Elektrode balkenförmig oder punktförmig ausgebildet ist, und/oder
**dass** sich die erste Elektrode und/oder die zweite Elektrode nichtparallel zu einer Erstreckungsrichtung des Behälters zwischen dem Bodenbereich und dem Mündungsbereich erstrecken.

7. System nach einem der vorangegangenen Ansprüche, **dadurch gekennzeichnet, dass** alle ersten Elektroden des Behälters über eine erste elektrische Leitungsanordnung elektrisch miteinander verbunden sind und/oder alle zweiten Elektroden des Behälters über eine zweite elektrische Leitungsanordnung elektrisch miteinander verbunden sind, wobei die erste elektrische Leitungsanordnung und/oder die zweite elektrische Leitungsanordnung vorzugsweise eine Isolatorschicht, vorzugsweise aus Siliziumdioxid, aufweist.

8. System nach einem der vorangegangenen Ansprüche, **dadurch gekennzeichnet, dass** das System eine Vielzahl von Behältern aufweist, wobei in mindestens zwei Behältern jeweils mindestens eine Vorrichtung angeordnet ist, wobei die ersten Elektroden zumindest eines Teils der Vielzahl der Behälter vorzugsweise mindestens eine erste gemeinsame Zuleitung aufweisen und/oder dass die zweiten Elektroden zumindest eines Teils der Vielzahl der Behälter mindestens eine zweite gemeinsame Zuleitung aufweisen, wobei die Behälter vorzugsweise in Spalten und Reihen angeordnet sind, wobei die ersten Elektroden der Behälter mindestens einer Reihe oder mindestens einer Spalte über mindestens eine von der ersten gemeinsamen Zuleitung erste abzweigende Zuleitung elektrisch miteinander verbunden sind und/oder die zweiten Elektroden der Behälter mindestens einer Reihe oder mindestens einer Spalte über mindestens eine von der zweiten gemeinsamen Zuleitung zweite abzweigende Zuleitung elektrisch miteinander verbunden sind, wobei vorzugsweise mindestens eine erste abzweigende Zuleitung mindestens einen Schalter zum elektrischen Trennen und Verbinden mit der mindestens einen ersten gemeinsamen Zuleitung aufweist und/oder dass mindestens eine zweite abzweigende Zuleitung mindestens einen Schalter zum elektrischen Trennen und Verbinden mit der mindestens einen zweiten gemeinsamen Zuleitung aufweist.

9. System nach einem der vorangegangenen Ansprüche, **dadurch gekennzeichnet, dass** das System weiter mindestens eine Vorrichtung zum Bereitstellen mindestens einer elektrischen Wechselspannung mit einer einstellbaren Frequenz aufweist, die mit der ersten Elektrode und/oder zweiten Elektrode elektrisch verbunden ist.

10. System nach einem der vorangegangenen Ansprüche, **dadurch gekennzeichnet, dass** das System weiter mindestens eine, vorzugsweise optische, Messeinrichtung zum Ermitteln einer Position mindestens einer Vorrichtung in mindestens einem Behälter aufweist, die an den Mündungsbereich des mindestens einen Behälters angeordnet werden kann, wobei die Behälter vorzugsweise auf einer beweglichen Transportvorrichtung angeordnet sind, und/oder
dass das System weiter ein Gerät zum Kryokonservieren mindestens einer Vorrichtung in mindestens einem Behälter aufweist.

11. Verfahren zum Charakterisieren und/oder Separieren mindestens einer biologischen Zelle, umfassen mindestens folgende Schritte:
- Bereitstellen mindestens eines Systems nach einem der vorangegangenen Ansprüche;
- Erzeugen eines ersten elektrischen Wechselfelds in dem Behälter mittels der ersten und zweiten Elektrode derart, dass die mindestens eine Vorrichtung in einem ersten Abstand von dem Bodenbereich entfernt angeordnet wird, wenn die mindestens eine biologische Zelle vital ist, und in einem zweiten Abstand von dem Bodenbereich entfernt angeordnet wird, wenn die mindestens eine biologische Zelle gestresst und/oder geschädigt ist, wobei der erste Abstand größer als der zweite Abstand ist, zum Separieren der mindestens einen biologischen Zelle;
- Ermitteln des Abstands der Vorrichtung von dem Bodenbereich zum Charakterisieren der mindestens einen biologischen Zelle.

12. Verfahren nach Anspruch 11, **dadurch gekennzeichnet, dass** das Verfahren weiter mindestens folgende Schritte aufweist:
- Erzeugen eines zweiten elektrischen Wechselfelds in dem Behälter mittels der ersten und zweiten Elektrode derart, dass die mindestens eine Vorrichtung in einem dritten Abstand von dem Bodenbereich entfernt angeordnet wird, wenn die mindestens eine biologische Zelle vital ist, und in einem vierten Abstand von dem Bodenbereich entfernt angeordnet wird, wenn die mindestens eine biologische Zelle gestresst und/oder geschädigt ist, wobei der vierte Abstand größer als der dritte Abstand ist; und
- Behandeln die Vorrichtung in dem Behälter, wobei das Behandeln vorzugsweise Kryokonservieren, Durchführen eines Substanztest, Durchführen von toxikologischen Tests und/oder Erfassen eines Zellwachstums umfasst, wobei das Verfahren zwischen dem Schritt: Erzeugen des zweiten elektrischen Wechselfelds, und dem Schritt: Behandeln, vorzugsweise weiter mindestens folgenden Schritt aufweist:
- Entfernen von Vorrichtungen, die in dem vierten Abstand angeordnet sind, aus dem jeweiligen Behälter.

13. Verfahren nach einem der Ansprüche 11 bis 12, **dadurch gekennzeichnet, dass** das Verfahren weiter mindestens folgenden Schritt aufweist:
- Bereitstellen mindestens einer kryokonservierten Vorrichtung in mindestens einem Behälter; und
- Erwärmen der mindestens einen kryokonservierten Vorrichtung und Erzeugen des ersten elektrischen Wechselfelds, vorzugsweise zumindest zeitweise mit einer Amplitude zwischen 4 V und 50 V, weiter vorzugsweise für weniger als 5 s, wobei die bereitgestellte kryokonservierte Vorrichtung vorzugsweise an dem Bodenbereich angeordnet ist.

14. Verfahren nach einem der Ansprüche 11 bis 13, **dadurch gekennzeichnet, dass** in mindestens zwei Behältern des Systems gleichzeitig ein elektrisches Wechselfeld mit gleicher Frequenz erzeugt wird, und/oder
dass das elektrische Wechselfeld dreidimensional und/oder rotationssymmetrisch ausgebildet ist und vorzugsweise ein elektrisches Gradientenfeld, weiter vorzugsweise ein nichtlineares elektrisches Gradientenfeld, aufweist, und/oder
dass die Vorrichtung aus dielektrischem Körper und der mindestens einen biologischen Zelle derart ausgebildet ist, dass sich die durch ein elektrisches Wechselfeld bei mindestens einer Wechselfrequenz erzeugte dielektrophoretische Krafteinwirkung auf die Vorrichtung verschwindet oder von einer negativen dielektrophoretischen Krafteinwirkung zu einer positiven dielektrophoretischen Krafteinwirkung ändert, wenn eine vorgegebene Anzahl an biologischen Zellen in der Vorrichtung überschritten wird, und/oder
dass der Schritt: Erzeugen eines ersten elektrischen Wechselfelds, dauerhaft durchgeführt wird, vorzugsweise bei einer Wechselfrequenz im Bereich von 100 kHz bis 1 GHz, weiter vorzugsweise im Bereich von 500 kHz bis 500 MHz, am meisten bevorzugt im Bereich von 5 MHz bis 100 MHz, wobei die mindestens eine biologische Zelle in dem dauerhaften ersten elektrischen Wechselfeld kultiviert wird, wobei die dauerhafte Durchführung des Schritts: Erzeugen eines ersten elektrischen Wechselfelds, vorzugsweise beendet wird, wenn eine vorgegebene Anzahl an biologischen Zellen kultiviert wurde, insbesondere, wenn sich die durch das elektrische Wechselfeld erzeugte dielektrophoretische Krafteinwirkung auf die Vorrichtung von einer negativen dielektrophoretischen Krafteinwirkung zu einer positiven dielektrophoretischen Krafteinwirkung ändert.

15. Verfahren nach einem der Ansprüche 11 bis 14, **dadurch gekennzeichnet, dass** das Verfahren weiter mindestens folgenden Schritt aufweist:
- Erfassen, vorzugsweise automatisiert, eines Wachstums der biologischen Zelle, und/oder dass der Schritt: Ermitteln des Abstands, mit einer Genauigkeit im Bereich von 0,001 µm bis 100 µm, vorzugsweise von 0,01 µm bis 10 µm durchgeführt wird, und/oder
dass das erste elektrische Wechselfeld durch Variieren einer Wechselfrequenz ermittelt wird und/oder durch das Ermitteln eines Rotationsspektrums der mindestens einen biologischen Zelle, wenn das elektrische Wechselfeld als ein rotierendes elektrisches Wechselfeld ausgebildet ist.
